# EUROPÄISCHE PATENTSCHRIFT

(11) **EP 1 783 200 B1**
(45) Veröffentlichungstag und Bekanntmachung des Hinweises auf die Patenterteilung: **11.12.2013**
(21) Anmeldenummer: 06022275.9
(22) Anmeldetag: 25.10.2006
(51) Int. Cl.: A23L 1/30

(54) **Strukturierte Lipidgemische mit CLA, omega-3 und/oder 6 Fettsäuren, und mittelkettigen Fettsäuren**
Structured lipid mixture with CLA, omega-3 and/or omega 6 fatty acids and medium chain fatty acids
Mélange de lipides structurés contentant d'acide linoléique conjugué, des acides gras omega-3 et/ou omega 6 et acides gras à chaîne moyenne

(30) Priorität: 03.11.2005 DE 102005052442
(43) Veröffentlichungstag der Anmeldung: 09.05.2007
(73) Patentinhaber: Cognis IP Management GmbH, 40589 Düsseldorf (DE)
(72) Erfinder: Schörken, Ulrich, 40591 Düsseldorf (DE); Bell, Doris, 40627 Düsseldorf (DE); Horlacher, Peter, 89287 Bellenberg (DE); Stuhlmann, Diana, 40627 Düsseldorf (DE); Meyer, Carolin, 40589 Düsseldorf (DE)

(56) Entgegenhaltungen:
- WO-A-03/043972
- US-A1- 2005 215 641
- TORRES, C.F., NETTEKOVEN, T.J., AND HILL, C.G.JR.: "Preparation of purified acylglycerols of eicosapentaenoic acid and docosahexaenoic acid and their re-esterification with conjugated linoleic acid" ENZYME AND MICROBIAL TECHNOLOGY, Bd. 32, 2003, Seiten 49-58, XP002419907 USSTONEHAM, MA
- RAES, K., HUYGHEBAERT, G., DE SMET, S., NOLLET, L., ARNOUTS, S., AND DEMEYER, D.: "The deposition of conjugated linoleic acids in eggs of laying hens fed diets varying in fat level and fatty acid profile" JOURNAL OF NUTRITION., Bd. 132, 2002, Seiten 182-189, XP002419908 USWISTAR INSTITUTE OF ANATOMY AND BIOLOGY, PHILADELPHIA, PA,
- TORRES C F ET AL: "ESTERIFICATION OF GLYCEROL WITH CONJUGATED LINOLEIC ACID AND LONG-CHAIN FATTY ACIDS FROM FISH OIL" JOURNAL OF THE AMERICAN OIL CHEMISTS' SOCIETY, AOCS PRESS, CHAMPAIGN, IL, US, Bd. 78, Nr. 11, November 2001 (2001-11), Seiten 1093-1098, XP001073021 ISSN: 0003-021X
- GARCIA, H.S., ARCOS, J.A., WARD, D.J., AND HILL, C.G. JR.: "Synthesis of glycerides containing n-3 fatty acids and conjugated linoleic acid by solvent-free acidolysis of fish oil" BIOTECHNOLOGY AND BIOENGINEERING., Bd. 70, Nr. 5, 2000, Seiten 587-591, XP002419909 USWILEY & SONS, HOBOKEN, NJ.
- GARCIA H S ET AL: "INTERESTERIFICATION (ACIDOLYSIS) OF BUTTERFAT WITH CONJUGATED LINOLEIC ACID IN A BATCH REACTOR" JOURNAL OF DAIRY SCIENCE, AMERICAN DAIRY SCIENCE ASSOCIATION, SAVOY, IL, US, Bd. 83, Nr. 3, März 2000 (2000-03), Seiten 371-377, XP000926920 ISSN: 0022-0302
- CHILLIARD, Y., FERLAY, A., MANSBRIDGE, R.M., AND DOREAU, M.: "Ruminant milk fat plasticity: nutritional control of saturates, polyunsaturated, trans and conjugated fatty acids." ANNALES DE ZOOTECHNIE., Bd. 49, 2000, Seiten 181-205, XP002419910 FRELSEVIER, PARIS
- HILLBRICK G ET AL: "MILKFAT CHARACTERISTICS AND FUNCTIONALITY: OPPORTUNITIES FOR IMPROVEMENT" AUSTRALIAN JOURNAL OF DAIRY TECHNOLOGY, DAIRY INDUSTRY ASSOCIATION OF AUSTRALIA, MELBOURNE, AU, Bd. 57, Nr. 1, April 2002 (2002-04), Seiten 45-51, XP001102103 ISSN: 0004-9433
- OSBORN, H.T, AND AKOH, C.C.: "Structured lipids-Novel fats with medical, nutraceutical and food applications" COMPREHENSIVE REVIEWS IN FOOD SCIENCE AND FOOD SAFETY, Bd. 3, 2002, Seiten 110-120, XP002419911 USINSTITUTE OF FOOD TECHNOLOGISTS, CHICAGO, IL

## Beschreibung

### Gebiet der Erfindung

Die Erfindung befindet sich auf dem Gebiet der Nahrungsmittelzusatzstoffe und - ergänzungsstoffe und betrifft strukturierte Lipidgemische, ein Verfahren zu ihrer Herstellung sowie ihre Verwendung im Bereich der Humanernährung, insbesondere zur Gewichtsregulierung.

### Stand der Technik

In den vergangenen Jahre haben spezielle Triglyceride mit C-Kettenlängen im Bereich von 6 bis 10 Kohlenstoffatomen, so genannte "Medium Chain Triglycerides" (MCT) an Bedeutung gewonnen, da sie im Bereich der Humanernährung die Aufnahme von Fettstoffen reduzieren und sowohl die Fettverbrennung als auch die Metabolisierungsgeschwindigkeit steigern. Auch in der praktischen Anwendung besitzen strukturierte Lipide gegenüber "normalen" natürlichen Lipiden eine große Zahl von Vorteilen, die jedoch stark vom Anwendungsgebiet abhängen. Typische Beispiele sind.
- Einstellung optimaler physikalischer Eigenschaften (z.B. bei Margarine, Confectionary Fats, CBE)
- Nachstellung eines speziellen Triglyceridmoleküls (z.B. bei OPO-Triglyceriden für die Säuglingsernährung)
- Hochkonzentrierte TGs für die Verabreichung von Wirkfettsäuren (z.B. Triglyceriden auf Basis konjugierter Linolsäure, Docosahexaencarbonsäure oder Eicosapentaensäure)
- Schnelle Energiezufuhr (MCTs und Lipide, die mittelkettige Fettsäuren enthalten)
- Lipide mit verringerter Kalorienzahl (z.B. Salatrim, Caprenin) und dietätische Öle mit Wirkung auf struktureller Basis (z.B. Enova-Öl, MCT basierte Lipide)
- Lipide mit beschleunigter Absorption (gezeigt für Lipide mit mittelkettigen Fettsäuren).

Aus den genannten Gründen wird in der Literatur immer wieder vorgeschlagen, konventionelle "weniger gesunde" Speiseöle, wie z.B. Sonnenblumen-, Oliven- oder Distelöl, gegen "besonders gesunde" MCT auszutauschen. Dies erweist sich in der Praxis allerdings als ausgesprochen schwierig, denn um von den vorteilhaften Eigenschaften der MCT zu profitieren, müsste ein durchschnittlicher Erwachsener im Mittel eine Dosis von 20 g/Tag aufnehmen -. Dies liegt jedoch bereits in der Größenordnung der durchschnittlichen Tagesverzehrmenge an Speiseölen, d.h., man dürfte dann keine weiteren Öle mehr zu sich nehmen.

Die komplexe Aufgabe, die der vorliegenden Erfindung daher zugrunde liegt, hat darin bestanden, ein Öl für den täglichen Gebrauch herzustellen, das
- eine gesunde Zusammensetzung enthält gemäß Empfehlungen von Ernährungsexperten in Bezug auf:
   - einen ausreichenden Anteil an essentiellen Fettsäuren wie z.B. Konjugierter Linolsäure (CLA) oder omega-3 bzw. omega-6 Fettsäuren,
   - ein ω-6 / ω-3 Verhältnis im Öl im Bereich von 5 : 1 bis 1 : 1,
   - einen hohen Anteil an Ölsäure von mehr als 20 Mol-% sowie
   - einen niedrigen Anteil an gesättigten und trans-Fettsäuren (die essentiellen Fettsäuren ausgenommen),
- zugleich einen Wirkcharakter besitzt in Bezug auf die Verringerung des Körperfettanteils begünstigt durch:
   - einen hohen Anteil direkt verstoffwechselbarer mittelkettiger Fettsäuren
   - eine empfohlene Dosis an wirksamen CLA-Isomeren
- und das besonders gut resorbiert werden kann durch einen hohen Anteil an strukturierten Triglyceriden mit ein oder zwei mittelkettiger Acylreste im Öl.

Eine weitere Anforderung an diese zu entwickelnden Produkte hat ferner darin bestanden, dass diese sich in ihrem physiko-chemischen Verhalten, z.B. was Trübungspunkt, Rauchtemperatur, Oxidationsstabilität und Viskosität angeht, ähnlich wie die bekannten Speiseöle verhalten. Zusätzlich bestand die Aufgabe darin, das strukturierte Lipid in einer sensorischen Qualität vergleichbar den üblichen Speiseölen herzustellen.

Eine letzte Aufgabe bestand schließlich darin, schonende Herstellverfahren für die strukturierten Lipide zu entwickeln, die die Herstellung qualitativ hochwertiger Produkte ermöglichen.

Torres, CF, Nettekoven, TJ und Hill, CGJR; Preparation of purified acylglycerols of eicosapentaenoic acid and docosahexaenoic acid and their reestrification with conjugated linoleic acid, Enzyme and microbial technology, Bd. 32, 2003, Seiten 49-58 offenbart ein Gemisch enthaltend Lipide mit 21 Mol-% CLA und 21 Mol-% Omega-3/Omega-6 Fettsäuren. Das Gemisch wird in den Nahrungsmitteln angewendet.

Raes, K, Huyghebaert, G, De Smet, S., Nollet, L., Arnouts, S und Demeyer, D.; The deposition of conjugated linoleic acids in eggs of laying hens fed diets varying in fat level and fatty acid profile, Journal of Nutrition, Bd. 132, 2002, Seiten 182-189 offenbart ein Gemisch enthaltend Lipide mit 5 Mol-% CLA und 19 Mol-% Omega-3/Omega-6 Fettsäuren. Das Lipidgemisch ist eine Nahrung.

Torres, CF et al.; Esterification of glycerol with conjugated linoleic acid and long-chain fatty acids from fish oil, Journal of the American oil chemist society, AOCS Press, Bd. 78, Nr. 11, November 2001, Seiten 1093-1098 offenbart ein Gemisch enthaltend Lipide mit 22 Mol-% CLA und 20 Mol-% Omega-3/Omega-6 Fettsäuren. Das Gemisch kann in den Nahrungsmitteln angewendet werden.

Garcia, HS, Arcos, JA, Ward, DJ und Hill, CGJR; Synthesis of glycerides containg n-3 fatty acids and conjugated linoleic acid by solvent-free acidolysis of fish oil, Biotechnology and Bioengineering, Bd. 70, Nr. 5, 2000, Seiten 587-591 offenbart ein Gemisch enthaltend Lipide mit 3-50 Mol-% CLA und 5-25 Mol-% Omega-3/Omega-6 Fettsäuren. Das Gemisch kann in den Nahrungsmitteln angewendet werden.

### Beschreibung der Erfindung

Gegenstand der Erfindung sind strukturierte Lipidgemische der Formel **(I),** 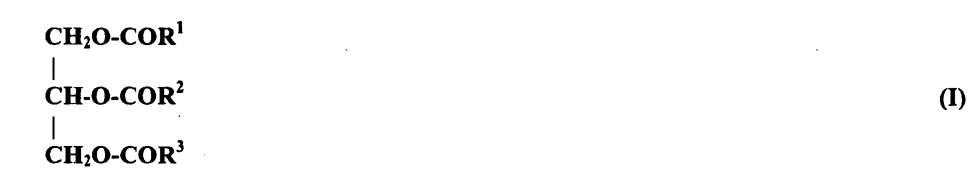 die sich dadurch auszeichnen, dass R¹CO, R²CO und R³CO unabhängig voneinander für
(i) lineare gesättigte Acylreste mit 6 bis 12 Kohlenstoffatomen oder
(ii) den Acylrest der konjugierten Linolsäure (CLA) und/oder einer omega-3 bzw. omega-6 Fettsäure (OF) stehen,
mit der Maßgabe, dass die Menge an CLA-Acylresten 3 bis 50 und vorzugsweise 5 bis 15 Mol-% ausmacht und/oder dass die Menge an OF-Acylresten mindestens 5 bis 25 und vorzugsweise 7 bis 15 Mol-% - jeweils bezogen auf die Menge an Acylresten - ausmacht.

Unter den Produkten der Formel (I), die statistische Verteilung der Fettsäuren in den drei möglichen Positionen aufweisen ("random"-Produkte), sind die Spezies der Formel (I), in der R¹CO und R³CO für lineare gesättigte Acylreste mit 6 bis 12 Kohlenstoffatomen und R²CO für den Acylrest der konjugierten Linolsäure (CLA) und/oder einer omega-3 bzw. omega-6 Fettsäure (OF) steht und die im Folgenden als "ABA-Typ" bezeichnet werden.

Im Hinblick auf die gewünschten Eigenschaften haben sich speziell solche Lipidgemische der Formel (I) als bevorzugt erwiesen, die sich durch die folgenden Strukturmerkmale - einzeln oder in beliebiger Kombination auszeichnen:
- Einen Gehalt an Triglyceriden, die sich entweder von einer mittelkettiger und zwei langkettigen Fettsäuren, oder von zwei mittelkettigen und einer langkettigen Fettsäure ableiten, von mindestens 60 Mol-%;
- Einen Gehalt von mehr als 20 Mol-% Ölsäure bezogen auf den Gehalt an langkettigen Fettsäuren und einen Gehalt von weniger als 20 Mo-1% gesättigten C₁₂-C₂₂-Fettsäuren sowie einen Gehalt von weniger als 2 Mol-% ungesättigten trans-Fettsäuren, wobei hiervon Anteile an CLA und OF ausgenommen sind.
- Einen Gehalt von mindestens 90 Mol-% der im Molekül gebundenen CLA, in Form des 9-cis, 11-trans Isomers oder des 10-trans, 12-cis Isomers oder Mischungen dieser beiden Isomere.
- Einen Gehalt an Acylresten, die, soweit sie sich von omega-3 bzw. omega-6 Fettsäuren ableiten, in einem ω-6 / ω-3 Verhältnis von 5 : 1 bis 1 : 1 vorliegen.

Überraschenderweise wurde gefunden, dass die erfindungsgemäßen strukturierten Lipide das gewünschte komplexe Anforderungsprofil bestens erfüllen und insbesondere neben ihren guten ernährungsphysiologischen Eigenschaften eine verbesserte Abbaubarkeit durch verbesserte Emulgierbarkeit und damit verbesserte Erreichbarkeit für Verdauungslipasen besitzen als zum Beispiel reines CLA-Triglycerid, was die Verfügbarkeit von CLA verbessert. Die verbesserte Verfügbarkeit der Wirkfettsäuren geht einher mit zusätzlichen Vorteilen in den physikalischen Produkteigenschaften. So wurden Lipide erhalten, die einen signifikant verbesserten Rauchpunkt um bis zu 25 °C im Vergleich zu entsprechenden Mischungen aus langkettigen und mittelkettigen Triglyceriden aufweisen und die eine deutlich erhöhte Oxidationsstabilität im Vergleich zu den eingesetzten Pflanzenölen und zum CLA-TG besitzen. Zusätzlich hatten die erfindungsgemäßen strukturierten Lipide verbesserte sensorische Eigenschaften sowohl in Bezug auf Geruch wie auch Geschmack im Vergleich zu den in der Synthese eingesetzten Pflanzenölen.

### Umesterung und Veresterung

Es soll an dieser Stelle vorsorglich darauf hingewiesen werden, dass unter dem Begriff konjugierte Linolsäure übliche technische Gemische von Stellungsisomeren zu verstehen sind, die unterschiedliche cis/trans-Verhältnisse aufweisen und gegebenenfalls in untergeordneten Mengen auch konventionelle Linolsäure sowie herstellungsbedingt andere Fettsäuren enthalten können. CLA wird üblicherweise durch basenkatalysierte Isomerisierung von Distelöl oder entsprechenden Alkylestern und nachfolgende enzymatische Hydrolyse hergestellt. Es hat sich dabei als vorteilhaft erwiesen, wenn die CLA eine bestimmte Spezifikation erfüllt, gemäß der der Acylrest wenigstens 30 Gew.-% t10, c12- Isomere, wenigstens 30 Gew.-% c9, t11-Isomere und in Summe weniger als 1 Gew.-% 8,10-, 11,13- und t,t-Isomere aufweist. Analoges gilt für das Triglycerid auf Basis konjugierter Linolsäure (CLA-TG). Entsprechende Produkte sind beispielsweise unter der Bezeichnung Tonalin^{®} CLA-80 und Tonalin^{®} CLA-TG im Handel. For die Herstellung pharmazeutischer Produkte hat es sich im Übrigen als besonders vorteilhaft erwiesen, wenn die CLA ausschließlich aus c10, t12 bzw. t9, c11 Isomeren besteht.

Omega-3 bzw. omega-6 Fettsäuren weisen üblicherweise 20 bis 28 Kohlenstoffatome und 4 bis 6 Doppelbindungen auf. Sie lassen sich beispielsweise aus marinen Quellen, speziell den verschiedenen Fischölen oder auf fermentativem Wege, z.B. mit Hilfe von Mikroalgen gewinnen. Die bekanntesten Vertreter sind DHA (Docohexaenoic acid) und EPA (Eicosapentaenoic acid).

Ein weiterer Gegenstand der vorliegenden Erfindung betrifft ein erstes Verfahren zur Herstellung strukturierter Lipidgemische der Formel (I), 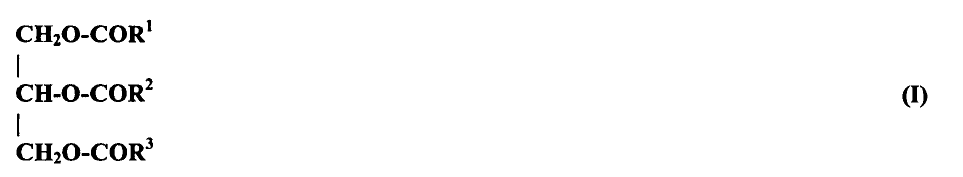 in der R¹CO, R²CO und R³CO unabhängig voneinander für
(i) lineare gesättigte Acylreste mit 6 bis 12 Kohlenstoffatomen oder
(ii) den Acylrest der konjugierten Linolsäure (CLA) und/oder einer omega-3 bzw. omega-6 Fettsäure (OF) stehen,
mit der Maßgabe, dass die Menge an CLA-Acylresten - 3 bis 50 und vorzugsweise 5 bis 15 Mol-% ausmacht und/oder dass die Menge an OF-Acylresten 5 bis 25 und vorzugsweise 7 bis 15 Mol-% - jeweils bezogen auf die Menge an Acylresten - ausmacht, welches sich dadurch auszeichnet, dass man (a) so genannte "medium chain triglycerides" (MCT) der Formel **(II),** 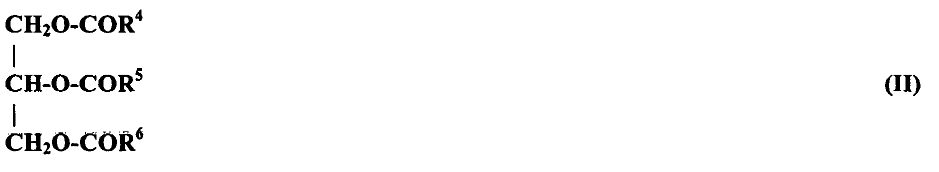 in der R⁴CO, R⁵CO und R⁶CO unabhängig voneinander für lineare gesättigte Acylreste mit 6 bis 12 Kohlenstoffatomen stehen, und (b) konjugierte Linolsäure (CLA) und/oder omega-3 bzw. omega-6 Fettsäuren (OF) und/oder Triglyceride auf Basis konjugierter Linolsäure (TG-CLA) bzw. omega-3 oder omega-6 Fettsäuren (TG-OF) einer enzymatischen Umesterung unterwirft.

Neben der Umesterung von MCT mit CLA bzw. CLA-TG können die neuen Stoffe auch über den Weg der Veresterung erhalten werden. Ein weiterer Gegenstand der vorliegenden Erfindung betrifft daher ein zweites Verfahren zur Herstellung strukturierter Lipidgemische der Formel **(I),** 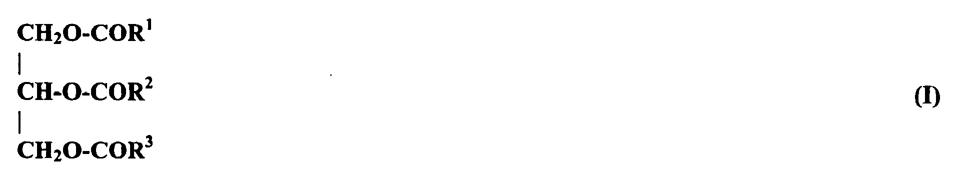 in der R¹CO, R²CO und R³CO
(i) unabhängig voneinander für lineare gesättigte Acylreste mit 6 bis 12 Kohlenstoffatomen oder
(ii) den Acylrest der konjugierten Linolsäure (CLA) oder einer omega-3 bzw. omega-6 Fettsäure (OF) stehen,
mit der Maßgabe, dass die Menge an CLA-Acylresten - 3 bis 50 und vorzugsweise 5 bis 15 Mol-% ausmacht und/oder dass die Menge an OF-Acylresten 5 bis 25 und vorzugsweise 7 bis 15 Mol-% - jeweils bezogen auf die Menge an Acylresten - ausmacht, welches sich dadurch auszeichnet, dass man (a) Gemische von Fettsäuren oder deren Estern der Formel **(III),**

**R⁷COOR⁸** (III)

in der R⁷CO für einen linearen gesättigten Acylrest mit 6 bis 12 Kohlenstoffatomen und R⁸ für Wasserstoff oder einen Alkylrest mit 1 bis 4 Kohlenstoffatomen und vorzugsweise Methyl steht, und (b) konjugierter Linolsäure (CLA) und/oder omega-3 bzw. omega-6 Fettsäuren einer enzymatischen Ver- bzw. Umesterung unterwirft.

Um die angestrebte Menge an CLA bzw. OF in die strukturierten Lipiden einzubauen, hat es sich des Weiteren als vorteilhaft erwiesen, wenn man die MCT bzw. Fettsäuren einerseits und die CLA und/oder OF bzw. deren Triglyceride andererseits bezogen auf die Fettsäureäquivalente im molaren Verhältnis 1 : 1 bis 20 : 1 einsetzt. Typische Beispiele hierfür sind Rapsöl, Sojaöl, Sonnenblumenöl, Distelöl, Olivenöl, Perillaöl, Borretschöl, Leinöl, Thunfischöl, Sardinenöl, Lachsöl, Makrelenöl sowie Algenöle und anderen mikrobielle Öle, die reich an polyungesättigten Fettsäuren sind. Dabei setzt man in der Regel - bezogen auf Fettsäureäquivalente - die MCT bzw. die Fettsäuren einerseits und die Öle andererseits im molaren Verhältnis 5 : 1 bis 1 : 5 und insbesondere 3 : 1 bis 1 : 3 ein. Die Bedingungen, unter denen die enzymatischen Reaktionen durchgeführt werden, sind dem Fachmann an sich bekannt und können, sofern dies durch die Beispiele nicht ohnehin ausreichen illustriert wird, von ihm aufgefunden und eingestellt werden, ohne dass es dazu erfinderischer Tätigkeit bedarf. Insbesondere hat sich sowohl für die Umesterung wie auch für die Veresterung eine Reaktionstemperatur im Bereich von 20 bis 70 und insbesondere 40 bis 60 °C sowie - unabhängig davon - eine Reaktionszeit von 2 bis 50, vorzugsweise 10 bis 25 Stunden als vorteilhaft erwiesen.

### Enzyme

Die Auswahl der Enzyme ist insofern kritisch, als dass sich hierdurch einerseits die regioselektivität als auch der Umsatz regulieren lassen. Grundsätzlich werden zur Herstellung der strukturierten Lipide, sei es über den Weg der Umesterung oder Veresterung, Enzyme vom Typ der Lipasen oder Esterasen benötigt. Vorzugsweise sind diese Lipasen *mikrobielle* Lipasen und dabei ausgewählt aus der Gruppe, die gebildet wird von *Rhizomucor miehei, Thermomyces lanugenosus* sowie *Candida antarctica B.* Des Weiteren hat es sich als vorteilhaft erwiesen, solche Lipasen einzusetzen, die in an sich bekannter Weise in immobilisierter Form vorliegen.

### Aufarbeitung der Reaktionsprodukte

Im Anschluss an die Umesterung bzw. Veresterung hat es sich als vorteilhaft erwiesen, nicht umgesetzte Fettsäuren, Ester und Monoglyceride aus den Reaktionsprodukten destillativ zu entfernen, beispielsweise bei Temperaturen unterhalb von 220 °C und Drücken von weniger als 1 mbar. Danach werden die Produkte üblicherweise einer Desodorierung und/oder Bleichung unter Verwendung von Bleicherde und/oder Aktivkohle unterworfen. Während und/oder nach der Reaktion kann man der Mischung im Übrigen für den Nahrungsmittelbereich zugelassene Antioxidantien beispielsweise in einer Konzentration von 100 - 2000 ppm zusetzen.

### Gewerbliche Anwendbarkeit

Ein weiterer Gegenstand der vorliegenden Erfindung ist somit auf die Verwendung der neuen strukturierten Lipidgemische als Nahrungsmittel oder Nahrungsergänzungsstoffe, als Bestandteil in pharmazeutischen Präparationen oder von Tierfutter gerichtet, wobei es sich bei diesen neuen Produkten beispielsweise um Speise- bzw. Diätspeiseöle sowie Brat- und Frittieröle und der gleichen handeln kann. Des Weiteren können die Lipide ein Bestandteil von Dressings, Mayonnaisen, Margarine, Milchprodukten, Säften, Back- und Süßwaren und der gleichen darstellen. Dabei können die Lipidgemische zur Umesterung mit gehärteten Fetten eingesetzt werden zur Erzeugung von Lipiden mit einstellbarem Solid Fat Content, Schmelz- und Kristallisationsverhalten. Die Lipide können des Weiteren in unterschiedlichsten Darreichungsformen, insbesondere auch in Form von Makro- oder Mikrokapseln eingesetzt werden. Eine weitere Darreichungsform ist in der Form stabiler Emulsionen mit Emulgatoren wie zum Beispiel Phospholipiden für einen Einsatz in pharmazeutischen Präparationen insbesondere zur parenteralen Ernährung.

Die Lipide im Allgemeinen können zur täglichen Ernährung eingesetzt werden und eignen sich für den kompletten Austausch der Nahrungsmittelfette. Die erfindungsgemäßen Lipide besitzen einen ausreichenden Gehalt an essentiellen Fettsäuren in einer ernährungsphysiologischvorteilhaften ω-6 / ω-3 Balance. Ein Zusatznutzen der Lipide sind die Wirkeffekte. Mittelkettige Fettsäuren versorgen den Körper mit schnell verfügbarer Energie ohne Körperfettmasse anzusetzen und CLA beeinflusst die Balance aus Körperfett und Muskelmasse positiv. Zusätzlich gewährleisten die strukturierten Lipide eine gute Verfügbarkeit der omega-3 bzw. omega-6 Fettsäuren sowie der CLA. Die erfindungsgemäßen strukturierten Lipide eignen sich daher zur Anwendung als dietätisches Öl und als Energielieferant in der Sportlerernährung sowie zur Ernährung von Menschen mit Erkrankungen des Fettstoffwechsels ebenso wie zur Herstellung eines Medikamentes zur Behandlung derartiger Erkrankungen.

Im Folgenden werden einige ausgewählte Ausführungsformen der vorliegenden Erfindung aufgeführt:
1. Gemisch enthaltend Lipide der Formel (I), 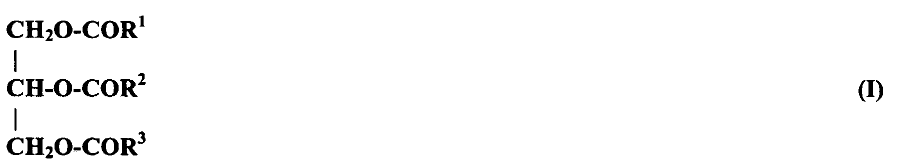 **dadurch gekennzeichnet,** dass R¹CO, R²CO und R³CO unabhängig voneinander ausgewählt sind aus der Gruppe bestehend aus
   (i) einem linearen gesättigten Acylrest mit 6 bis 12 Kohlenstoffatomen,
   (ii) dem Acylrest der konjugierten Linolsäure (CLA)
   (iii) dem Acylrest einer omega-3 Fettsäure (OF) und
   (iv) dem Acylrest einer omega-6 Fettsäure (OF),
   mit der Maßgabe, dass die Menge an CLA-Acylresten in dem Gemisch 3 bis 50 Mol-% und die Menge an OF-Acylresten in dem Gemisch 5 bis 25 Mol-% - jeweils bezogen auf die Menge aller Acylreste in dem Gemisch - ausmacht.
2. Gemisch nach Ausführungsform 1, **dadurch gekennzeichnet,** dass R¹CO und R³CO für lineare gesättigte Acylreste mit 6 bis 12 Kohlenstoffatomen und R²CO für den Acylrest der konjugierten Linolsäure (CLA) und/oder einer omega-3 bzw. omega-6 Fettsäure (OF) steht (ABA-Typ).
3. Gemisch nach den Ausführungsformen 1 oder 2, dadurch gekennzeichnet, dass es einen Gehalt an Triglyceriden, die sich entweder von einer mittelkettiger und zwei langkettigen Fettsäuren, oder von zwei mittelkettigen und einer langkettigen Fettsäure ableiten, von mindestens 60 Mol-% aufweist.
4. Gemisch nach einer der Ausführungsformen 1 bis 3, **dadurch gekennzeichnet,** es einen Gehalt von mehr als 20 Mol-% Ölsäure bezogen auf den Gehalt an langkettigen Fettsäuren und einen Gehalt von weniger als 20 Mo-1% gesättigten C₁₂-C₂₂-Fettsäuren sowie einen Gehalt von weniger als 2 Mol-% ungesättigten trans-Fettsäuren aufweist, wobei hiervon Anteile an CLA und OF ausgenommen sind.
5. Gemisch nach einer der Ausführungsformen 1 bis 4, dadurch gekennzeichnet, dass mindestens 90 Mol-% der im Molekül gebundenen CLA entweder das cis9,trans11 Isomer oder das 10trans,cis 12 Isomer oder Mischungen dieser beiden Isomere darstellen.
6. Gemisch nach nach einer der Ausführungsformen 1 bis 5, dadurch gekennzeichnet, dass die Acylreste, soweit sie sich von omega-3 bzw. omega-6 Fettsäuren ableiten, in einem ω -6/ω-3 Verhältnis von 5 : 1 bis 1 : 1 vorliegen
7. Verfahren zur Herstellung des Gemisches nach Ausführungsform 1, dadurch
   gekennzeichnet, dass man
   (a) so genannte "medium chain triglycerides" (MCT) der Formel **(II),** 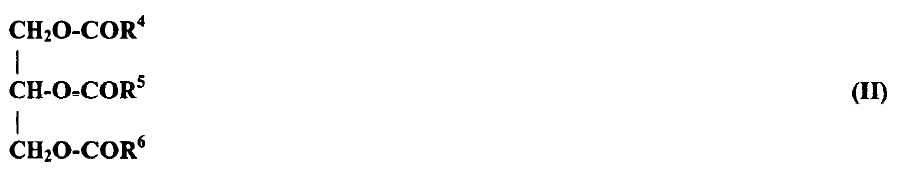 wobei R⁴CO, R⁵CO und R⁶CO unabhängig voneinander jeweils für einen linearen gesättigten Acylrest mit 6 bis 12 Kohlenstoffatomen stehen, und
   (b) konjugierte Linolsäure (CLA) und/oder omega-3 Fettsäuren (OF) und/oder omega-6 Fettsäuren (OF) und/oder Triglyceride auf Basis konjugierter Linolsäure (TG-CLA) und/oder Triglyceride auf Basis von omega-3 oder von omega-6 Fettsäuren (TG-OF) einer enzymatischen Umesterung unterwirft.
8. Verfahren zur Herstellung des Gemisches nach Ausführungsform 1, dadurch gekennzeichnet, dass man
   (a) Gemische von Fettsäuren oder deren Estern der Formel (III),

      **R⁷COOR⁸** **(III)**

      in der R⁷CO für einen linearen gesättigten Acylrest mit 6 bis 12 Kohlenstoffatomen und R⁸ für Wasserstoff oder einen Alkylrest mit 1 bis 4 Kohlenstoffatomen steht, und
   (b) konjugierter Linolsäure (CLA) und/oder omega-3 und/oder omega-6 Fettsäuren einer enzymatischen Ver- bzw. Umesterung unterwirft.
9. Verfahren nach einer der Ausführungsformen 7 oder 8, dadurch gekennzeichnet, dass man die MCT bzw. die Fettsäuren einerseits und die CLA und/oder OF bzw. deren Triglyceride andererseits bezogen auf die Fettsäureäquivalente im molaren Verhältnis 1 : 1 bis 20 : 1 einsetzt.
10. Verfahren nach einer der Ausführungsformen 7 bis 9, **dadurch gekennzeichnet,** dass man die Umesterung bzw. Veresterung in Gegenwart von pflanzlichen oder marinen oder mikrobiellen Öle oder deren Gemischen durchführt.
11. Verfahren nach Ausführungsform 10, **dadurch gekennzeichnet,** dass man Öle einsetzt, die ausgewählt sind aus der Gruppe, die gebildet wird von Rapsöl, Sojaöl, Sonnenblumenöl, Distelöl, Olivenöl, Thunfischöl, Sardinenöl, Lachsöl, Makrenenöl sowie Algenölen und anderen mikrobiellen Ölen, die reich an polyungesättigten Fettsäuren sind.
12. Verfahren nach einer der Ausführungsformen 10 oder 11, dadurch gekennzeichnet, dass man - bezogen auf Fettsäureäquivalente - die MCT bzw. die Fettsäuren einerseits und die Öle andererseits im molaren Verhältnis 5 : 1 bis 1 : 5 einsetzt.
13. Verfahren nach einer der Ausführungsformen 7 bis 12, dadurch gekennzeichnet, dass man die enzymatische Umesterung bzw. Veresterung in Gegenwart mindestens einer Lipase oder Esterase durchführt.
14. Verfahren nach einer der Ausführungsformen 7 bis 13, dadurch gekennzeichnet, dass man Lipasen einsetzt, die ausgewählt sind aus der Gruppe der mikrobiellen Lipasen
15. Verfahren nach einer der Ausführungsformen 7 bis 14, **dadurch gekennzeichnet,** dass man mikrobielle Lipasen einsetzt, die ausgewählt sind aus der Gruppe, die gebildet wird von *Rhizomucor, Rhizopus, Thermomyces, Pseudomonas* sowie *Candida.*
16. Verfahren nach einer der Ausführungsformen 7 bis 15, **dadurch gekennzeichnet,** dass man immobilisierte Lipasen einsetzt.
17. Verfahren nach einer der Ausführungsformen 7 bis 16, **dadurch gekennzeichnet,** dass man die enzymatische Umesterung bzw. Veresterung bei Temperaturen im Bereich von 20 bis 70 °C durchführt.
18. Verfahren nach einer der Ausführungsformen 7 bis 17, dadurch gekennzeichnet, dass man die enzymatische Umesterung bzw. Veresterung über einen Zeitraum von 2 bis 50 Stunden durchführt.
19. Verfahren nach einer der Ausführungsformen 7 bis 18, **dadurch gekennzeichnet,** dass man nicht umgesetzte Fettsäuren, Ester und Monoglyceride aus den Reaktionsprodukten destillativ entfernt.
20. Verfahren nach einer der Ausführungsformen 7 bis 19, dadurch gekennzeichnet, dass man die Reaktionsprodukte anschließend einer Desodorierung und/oder Bleichung unterwirft.
21. Verfahren nach einer der Ausführungsformen 7 bis 21, dadurch gekennzeichnet, dass man während und/oder nach der Reaktion der Mischung für den Nahrungsmittelbereich zugelassene Antioxidantien zusetzt.
22. Verwendung des Gemisches nach Ausführungsform 1 als Nahrungsmittel, Nahrungsergänzungsmittel, pharmazeutische Präparation oder Tierfutter.
23. Verwendung nach Ausführungsform 22, **dadurch gekennzeichnet,** dass die Nahrungsmittel Speise- bzw. Diätspeiseöle, Brat- und Frittieröle und der gleichen darstellen.
24. Verwendung nach Ausführungsform 22, **dadurch gekennzeichnet,** dass das Gemisch Bestandteil von Dressings, Mayonnaisen, Margarine, Milchprodukten, Säften, Back- und Süsswaren und der gleichen darstellt.
25. Verwendung nach Ausführungsform 22, dadurch gekennzeichnet, dass man das Gemisch zur Umesterung mit gehärteten Fetten und zur Erzeugung von Lipiden mit einstellbarem Solid Fat Content, Schmelz- und Kristallisationsverhalten einsetzt.
26. Verwendung nach Ausführungsform 22, **dadurch gekennzeichnet,** dass man das Gemisch zur parenteralen Ernährung einsetzt.
27. Verwendung nach einem der Ausführungsformen 22 bis 26, **dadurch gekennzeichnet,** dass man das Gemisch in Form von Emulsionen mit Phospholipiden oder Kapseln einsetzt.
28. Verwendung nach einer der Ausführungsformen 22 bis 27, **dadurch gekennzeichnet,** dass man die Lipidgemische zur Versorgung mit konjugierter Linolsäure und/oder omega3 bzw. omega-6 Fettsäuren und zur Reduktion von Fettgewebe einsetzt.
29. Verwendung nach einer der Ausführungsformen 22 bis 28, **dadurch gekennzeichnet,** dass man die Lipidgemische als dietätisch wirksame Öle einsetzt.
30. Verwendung nach einer der Ausführungsformen 22 bis 29, **dadurch gekennzeichnet,** dass man die Lipidgemische als schneller Energielieferant und zum Aufbau von Muskelmasse in der Sportlerernährung einsetzt.
31. Verwendung des Gemisches nach Ausführungsform 1 zur Herstellung eines Medikamentes zur Behandlung von Erkrankungen des Fettstoffwechsels.

### Beispiele

### Beispiel 1

### Herstellung von strukturierten Lipiden durch enzymatische Umesterung

In vier Ansätzen wurden jeweils 25 g MCT (Middle Chain Triglyceride, Myritol^{®} 314, Cognis Deutschland GmbH & Co. KG), 5 g CLA-Triglycerid (Tonalin^{®} TG 80, Cognis Deutschland GmbH & Co. KG) sowie 20 g Rapsöl eingewogen. Zu den Ansätzen 1 und 3 wurden jeweils 2,5 g immobilisierte *Rhizomucor miehei* Lipase und zu den Ansätzen 2 und 4 jeweils 2,5 g immobilisierte *Thermomyces lanugenosus* Lipase gegeben. Die Ansätze 1 und 2 wurden bei 45 °C und die Ansätze 3 und 4 bei 60 °C unter Schütteln inkubiert. Nach 5 h bzw. 29 h (nur von Ansätzen 1 + 2) wurden jeweils Proben entnommen und gaschromatographisch analysiert. Die Ergebnisse sind in Tabelle 1 zusammengefasst. Angegeben ist die Zusammensetzung in GC-Flächen-% sowie die Säurezahl.

**Tabelle 1**

| **Zusammensetzung des Reaktionsproduktes** | | | | | | |
|---|---|---|---|---|---|---|
| **Zusammensetzung** | **1a** | **1b** | **1c** | **1d** | **1e** | **1f** |
| Ansatz Nr. | 1 | 1 | 2 | 2 | 3 | 4 |
| Reaktionszeit [h] | 5 | 29 | 5 | 29 | 5 | 5 |
| *Glyceridverteilung [Flächen-%]* | | | | | | |
| - FS (mittelkettig) | 2,6 | 2,6 | 2,4 | 2,3 | 2,7 | 2,4 |
| - FS (langkettig) | 1,7 | 2,1 | 2,0 | 2,0 | 2,0 | 2,0 |
| - MG (mittelkettig) | 0 | 0 | 0 | 0 | 0 | 0 |
| - MG (langkettig) | 0 | 0 | 0 | 0 | 0 | 0 |
| - DG (2x mittelkettig) | 4,2 | 3,6 | 3,4 | 3,2 | 4,0 | 3,4 |
| - DG (1x mittel-, 1x langkettig) | 2,0 | 1,9 | 1,5 | 1,4 | 2,0 | 1,7 |
| - DG (2x langkettig) | 0,4 | 0,5 | 0,5 | 0,6 | 0,9 | 0,3 |
| - TG (3x mittelkettig) | 34,4 | 23,9 | 24,7 | 21,4 | 29,7 | 21,6 |
| - TG (2x mittel-, 1x langkettig) | 23,1 | 35,1 | 37,8 | 39,3 | 28,6 | 39,3 |
| - TG (1x mittel-, 2x langkettig) | 15,3 | 24,3 | 23,0 | 26,1 | 21,1 | 25,3 |
| - TG (3x langkettig) | 16,3 | 6,0 | 4,7 | 3,7 | 9,2 | 4,1 |
| Säurezahl | 7 | 7 | 7 | 8 | 8 | 7 |

Die Beispiele zeigen, dass die Umesterung mit beiden immobilisierten Lipasen sowohl bei 45°C als auch bei 60 °C mit ausgezeichneten Ausbeuten abläuft.

### Beispiel 2

### Herstellung von strukturierten Lipden durch enzymatische Umesterung mit destillativer Produktreinigung

In einen Kolben wurden 750 g MCT, 150 g CLA-Triglycerid sowie 600 g Rapsöl eingewogen. Der Ansatz wurde mit Stickstoff überschichtet, mit 75 g immobilisierter *Thermomyces lanugenosus* Lipase versetzt und für 24 h unter Schütteln inkubiert. Nach 5 h bzw. 24 h wurden jeweils Proben entnommen und gaschromatographisch sowie auf Säurezahl analysiert. Nach 24 h wurde das Enzym über Filtration vom Rohprodukt entfernt. Das Rohprodukt wurde bei 200 °C und einem Vakuum vom 0,4 mbar über eine Kurzwegdestillation aufgereinigt. Anschließend wurden das Sumpfprodukt sowie das Destillat gaschromatographisch und nasschemisch analysiert. Dabei wurden die Verteilung der Glyceride sowie das Fettsäurespektrum analysiert, sowie die fettchemischen Kennzahlen des Sumpfproduktes bestimmt. Das Sumpfprodukt wurde mit 50 Gew.-% Natriumhydroxid (zweifache molare Menge bezogen auf freie Fettsäuren) bei 80 °C für 15 min raffiniert, 15 min bei 100 °C mit 2 % Bleicherde Tonsil behandelt und danach mit 5 Gew.-% Wasser gewaschen. Anschließend wurde die Wasserphase abgetrennt und der Rückstand im Vakuum getrocknet. Das fertige Produkt wurde zur Stabilisierung mit 500 ppm gemischten Tocopherolen (Covi-ox T-90) versetzt. Die Ergebnisse sind in Tabelle 2 zusammengefasst. Angegeben ist die Zusammensetzung in GC-Flächen-% sowie die Säurezahl. In Tabelle 3 sind die Säurezahlen sowie die Farbzahlen nach Lovibond und Gardner vor und nach der Raffination und Bleiche angegeben.

**Tabelle 2**

| **Zusammensetzung des Reaktionsproduktes** | | | | |
|---|---|---|---|---|
| **Zusammensetzung** | **2a** | **2b** | **2c** | **2d** |
| Produkt | 5 h Synthese | 24 h Synthese | Destillation Sumpf | Destillation Destillat |
| *Glyceridverteilung [Flächen-%]* | | | | |
| - FS (mittelkettig) | 1,5 | 1,3 | 0,1 | 31,6 |
| - FS (langkettig) | 0,7 | 0,8 | 0,7 | 16,6 |
| - MG (mittelkettig) | 0 | 0 | 0 | 0,8 |
| - MG (langkettig) | 0 | 0 | 0 | 0 |
| - DG (2x mittelkettig) | 2,3 | 2,0 | 1,4 | 16,8 |
| - DG (1x mittel-, 1x langkettig) | 0,5 | 1,0 | 0,7 | 0,4 |
| - DG (2x langkettig) | 0,3 | 0,5 | 0,4 | 0 |
| - TG (3x mittelkettig) | 33,7 | 21,6 | 21,6 | 31,5 |
| - TG (2x mittel-, 1x langkettig) | 30,2 | 40,9 | 41,7 | 2,2 |
| - TG (1x mittel-, 2x langkettig) | 16,0 | 27,5 | 28,7 | 0,1 |
| - TG (3x langkettig) | 14,8 | 4,4 | 4,7 | 0 |

| *Fettsäurespektrum* | | | | |
|---|---|---|---|---|
| - C8 | | 30,0 | 29,7 | 49,9 |
| - C10 | | 13,5 | 13,9 | 19,0 |
| - C16:0 | | 2,4 | 2,4 | 2,2 |
| - C18:0 | | 1,2 | 1,2 | 0,8 |
| - C18:1 | | 29,8 | 39,9 | 17,4 |
| - C18:2 | | 9,5 | 9,4 | 4,3 |
| - C18:3 | | 4,0 | 3,9 | 1,7 |
| - C9,t11 CLA | | 4,9 | 4,8 | 2,4 |
| - t10,c12 CLA | | 4,7 | 4,7 | 2,2 |

| *Nasschemische Kennzahlen* | | | | |
|---|---|---|---|---|
| Säurezahl | 5 | 5 | 1,3 | 137 |
| Hydroxylzahl | | | 6,4 | |
| Iodzahl | | | 57 | |
| Verseifungszahl | | | 261 | |
| Peroxidzahl | | | 5,0 | |

**Tabelle 3**

| **Beschreibung des Reaktionsproduktes** | | | |
|---|---|---|---|
| **Farbzahlen** | **Sumpfprodukt** nach Destillation | **Produkt** gebleicht, raffiniert | **Rapsöl** |
| Gardner | 3,4 | 1,0 | 1,8 |
| Lovibond 5 ¼ | Ly 24,0; Lr 2,8 | Ly 7,8; Lr 1,0 | Ly 12,0; Lr 1,4 |
| Säurezahl | 1,3 | 0,5 | 0,4 |

Die Beispiele zeigen, dass über enzymatische Umesterung und destillative Reinigung strukturierte Lipide hoher Qualität hergestellt werden können.

### Beispiel 3

### Herstellung strukturierter Lipide durch enzymatische Umesterung mit destillativer Produktreinigung

In einen Kolben wurden 450 g MCT, 150 g CLA-Triglycerid sowie 900 g Rapsöl vorgelegt. Der Ansatz wurde mit Stickstoff überschichtet, mit 75 g immobilisierter *Thermomyces lanugenosus* Lipase versetzt und für 24 h unter Schütteln inkubiert. Nach 5 h bzw. 24 h wurden jeweils Proben entnommen und gaschromatographisch sowie auf Säurezahl analysiert. Nach 24 h wurde das Enzym über Filtration vom Rohprodukt entfernt. Das Rohprodukt wurde bei 200 °C und einem Vakuum vom 0,4 mbar über eine Kurzwegdestillation aufgereinigt. Das Sumpfprodukt sowie das Destillat wurden gaschromatographisch und nasschemisch analysiert. Dabei wurden die Verteilung der Glyceride sowie das Fettsäurespektrum analysiert, sowie die fettchemischen Kennzahlen des Sumpfproduktes bestimmt. Das Sumpfprodukt wurde mit 50 Gew.-% Natriumhydroxid (zweifache molare Menge bezogen auf freie Fettsäuren) bei 80 °C für 15 min raffiniert, 15 min bei 100 °C mit 2 Gew.-% Bleicherde Tonsil behandelt und schließlich mit 5 Gew.-% Wasser gewaschen. Anschließend wurde die Wasserphase abgetrennt und das Produkt im Vakuum getrocknet. Das fertige Produkt wurde zur Stabilisierung mit 500 ppm gemischten Tocopherolen (Covi-ox T-90) versetzt. Die Ergebnisse sind in Tabelle 4 zusammengefasst. Angegeben ist die Zusammensetzung in GC-Flächen-% sowie die Säurezahl. In Tabelle 5 sind die Säurezahlen sowie die Farbzahlen nach Lovibond und Gardner vor und nach der Raffination und Bleiche angegeben.

**Tabelle 4**

| **Zusammensetzung des Reaktionsproduktes** | | | | |
|---|---|---|---|---|
| **Zusammensetzung** | **3a** | **3b** | **3c** | **3d** |
| Produkt | 5 h Synthese | 24 h Synthese | Destillation Sumpf | Destillation Destillat |
| *Glyceridverteilung [Flächen-%]* | | | | |
| - FS (mittelkettig) | 1,0 | 0,9 | 0 | 29,2 |
| - FS (langkettig) | 0,8 | 1,1 | 0,7 | 35,1 |
| - MG (mittelkettig) | 0 | 0 | 0 | 0,9 |
| - MG (langkettig) | 0 | 0 | 0 | 0 |
| - DG (2x mittelkettig) | 0,9 | 0,7 | 0,7 | 13,4 |
| - DG (1x mittel-, 1x langkettig) | 0,5 | 0,9 | 0,5 | 0,9 |
| - DG (2x langkettig) | 0,5 | 1,0 | 0,5 | 0 |
| - TG (3x mittelkettig) | 16,8 | 8,5 | 9,5 | 18,3 |
| - TG (2x mittel-, 1x langkettig) | 25,3 | 30,8 | 32,5 | 2,1 |
| - TG (1x mittel-, 2x langkettig) | 26,3 | 41,3 | 43,2 | 0,1 |
| - TG (3x langkettig) | 27,9 | 14,8 | 12,4 | 0 |

| *Fettsäurespektrum* | | | | |
|---|---|---|---|---|
| - C8 | | 19,9 | 20,5 | 35,0 |
| - C10 | | 8,8 | 9,2 | 14,2 |
| - C16:0 | | 3,0 | 3,0 | 3,7 |
| - C18:0 | | 1,4 | 1,3 | 1,2 |
| - C18:1 | | 39,3 | 38,5 | 29,4 |
| - C18:2 | | 12,7 | 12,6 | 7,5 |
| - C18:3 | | 5,5 | 5,5 | 3,2 |
| - C9,t11 CLA | | 4,8 | 4,7 | 3,0 |
| - t10,c12 CLA | | 4,7 | 4,6 | 2,9 |

| *Nasschemische Kennzahlen* | | | | |
|---|---|---|---|---|
| Säurezahl | 4 | 5 | 1,4 | 168 |
| Hydroxylzahl | | | 6,2 | |
| Iodzahl | | | 66 | |
| Verseifungszahl | | | 239 | |
| Peroxidzahl | | | 9,4 | |

**Tabelle 5**

| **Beschreibung des Reaktionsproduktes** | | | |
|---|---|---|---|
| **Farbzahlen** | **Sumpfprodukt** nach Destillation | **Produkt** gebleicht, raffiniert | **Rapsöl** |
| Gardner | 3,6 | 1,1 | 1,8 |
| Lovibond 5 ¼ | Ly 26,0; Lr 3,1 | Ly 8,2; Lr 0,9 | Ly 12,0; Lr 1,4 |
| Säurezahl | 1,4 | 0,4 | 0,4 |

Die Beispiele zeigen, dass über enzymatische Umesterung und destillative Reinigung strukturierte Lipide hoher Qualität hergestellt werden können.

### Beispiel 4

### Herstellung strukturierter Lipide durch enzymatische Synthese

In zwei Ansätzen wurden jeweils 30 g Caprylsäure, 12 g Caprinsäure, 9 g 80 Gew.-%ige CLA Fettsäure (Tonalin^{®} CLA 80; Cognis Deutschland GmbH & Co. KG), 9 g Glycerin sowie 40 g Rapsöl eingewogen. Zu Ansatz 1 wurden 5 g immobilisierte *Candida antarctica B* Lipase und zu Ansatz 2 5 g immobilisierte *Rhizomucor miehei* Lipase gegeben. Die Ansätze wurden bei 60 °C und einem Vakuum von 20 mbar unter Rühren und Stickstoff-Begasung inkubiert. Nach 6 h und 24 h wurden jeweils Proben entnommen und gaschromatographisch analysiert. Die Ergebnisse sind in Tabelle 6 zusammengefasst. Angegeben ist die Zusammensetzung in GC-Flächen-% sowie die Säurezahl (gemessen nach 50 h).

**Tabelle 6**

| **Zusammensetzung des Reaktionsproduktes** | | | | |
|---|---|---|---|---|
| **Zusammensetzung** | **4a** | **4b** | **4c** | **4d** |
| Ansatz Nr. | 1 | 1 | 2 | 2 |
| Reaktionszeit [h] | 6 | 24 | 6 | 24 |

| *Glyceridverteilung [Flächen-%]* | | | | |
|---|---|---|---|---|
| - FS (mittelkettig) | 11,3 | 5,9 | 16,9 | 10,4 |
| - FS (langkettig) | 7,2 | 5,1 | 10,3 | 7,8 |
| - MG (mittelkettig) | 0 | 0 | 0 | 0 |
| - MG (langkettig) | 0 | 0 | 0 | 0 |
| - DG (2x mittelkettig) | 6,9 | 0,5 | 12,8 | 8,1 |
| - DG (1x mittel-, 1x langkettig) | 6,7 | 0 | 21,1 | 2,5 |
| - DG (2x langkettig) | 1,3 | 0,7 | 1,4 | 0,9 |
| - TG (3x mittelkettig) | 20,3 | 21,6 | 3,2 | 16,5 |
| - TG (2x mittel-, 1x langkettig) | 20,4 | 34,8 | 13,6 | 27,8 |
| - TG (1x mittel-, 2x langkettig) | 11,7 | 22,9 | 15,2 | 21,9 |
| - TG (3x langkettig) | 14,2 | 8,4 | 5,0 | 4,1 |
| Säurezahl | 21 | | 20 | |

Die Beispiele zeigen, dass man sowohl mit immobilisierter *Candida B* Lipase wie auch mit immobilisierter *Rhizomucor miehei* Lipase eine Synthese mit gleichzeitiger Umesterung erreicht. Die *Candida B* Lipase zeigt dabei eine bessere Syntheseaktivität, während die *Rhizomucor miehei* Lipase eine bessere Umesterungsaktivität besitzt.

### Beispiel 5

### Herstellung strukturierter Lipide durch enzymatische Synthese mit destillativer Produktreinigung

In einen beheizbaren Doppelmantelreaktor wurden 450 g Caprylsäure, 180 g Caprinsäure, 135 g 80 Gew.-%ige CLA Fettsäure (Tonalin^{®} CLA 80), 142,5 g Glycerin sowie 900 g Rapsöl eingewogen. Zum Ansatz wurden 37,5 g immobilisierte *Candida antarctica B* Lipase und 37,5 g immobilisierte *Rhizomucor miehei* Lipase gegeben und der Ansatz für 48 h bei 60 °C unter Rühren und Stickstoffbegasung bei einem Vakuum von 5 mbar inkubiert. Nach 5 h, 24 h bzw. 48 h wurden jeweils Proben entnommen und gaschromatographisch sowie auf Säurezahl analysiert. Nach 48 h wurde das Enzym über Filtration vom Rohprodukt entfernt, welches anschließend bei 200 °C und einem Vakuum vom 0,4 mbar über eine Kurzwegdestillation aufgereinigt wurde. Das Sumpfprodukt sowie das Destillat wurden gaschromatographisch sowie nasschemisch analysiert und die Verteilung der Glyceride sowie das Fettsäurespektrum analysiert, sowie die fettchemischen Kennzahlen des Sumpfproduktes bestimmt. Das Sumpfprodukt wurde mit 50 Gew.-% Natriumhydroxid (zweifache molare Menge bezogen auf freie Fettsäuren) bei 80 °C für 15 min raffiniert und 15 min bei 100 °C mit 2 % Bleicherde Tonsil behandelt. Anschließend wurde das Produkt mit 5 Gew.-% Wasser gewaschen, die Wasserphase separiert und der Rückstand im Vakuum getrocknet. Das fertige Produkt wurde zur Stabilisierung mit 500 ppm gemischten Tocopherolen (Covi-ox T-90) versetzt. Die Ergebnisse sind in Tabelle 7 zusammengefasst. Angegeben ist die Zusammensetzung in GC-Flächen-% sowie die Säurezahl. In Tabelle 8 sind die Säurezahlen sowie die Farbzahlen nach Lovibond und Gardner vor und nach der Raffination und Bleiche angegeben.

**Tabelle 7**

| **Zusammensetzung des Reaktionsproduktes** | | | | | |
|---|---|---|---|---|---|
| **Zusammensetzung** | **5a** | **5b** | **5c** | **5d** | **5e** |
| Produkt | 5 h Synthese | 24 h Synthese | 48 h Synthese | Destillation Sumpf | Destillation Destillat |
| *Glyceridverteilung [Flächen-%]* | | | | | |
| - FS (mittelkettig) | 16,4 | 4,0 | 3,8 | 0 | 18,8 |
| - FS (langkettig) | 8,7 | 4,1 | 3,1 | 0,2 | 22,7 |
| - MG (mittelkettig) | 0 | 0 | 0 | 0 | 0,3 |
| - MG (langkettig) | 0 | 0 | 0 | 0 | 0 |
| - DG (2x mittelkettig) | 13,6 | 0,5 | 0,2 | 0 | 3,7 |
| - DG (1x mittel-, 1x langkettig) | 9,9 | 0,8 | 0 | 0,2 | 0,3 |
| - DG (2x langkettig) | 0,9 | 0,4 | 0,6 | 0,6 | 0 |
| - TG (3x mittelkettig) | 10,1 | 17,4 | 18,3 | 12,9 | 49,6 |
| - TG (2x mittel-, 1x langkettig) | 12.6 | 39,3 | 39,9 | 45,9 | 4,5 |
| - TG (1x mittel-, 2x langkettig) | 10,8 | 28,3 | 29,3 | 34,7 | 0,1 |
| - TG (3x langkettig) | 16,5 | 5,2 | 4,8 | 5,5 | 0 |

| *Fettsäurespektrum* | | | | | |
|---|---|---|---|---|---|
| - C8 | | | 30,9 | 27,2 | 56,0 |
| - C10 | | | 13,9 | 13,8 | 16,9 |
| - C16:0 | | | 2,5 | 2,5 | 1,4 |
| - C18:0 | | | 1,2 | 1,2 | 0,6 |
| - C18:1 | | | 29,4 | 31,7 | 15,1 |
| - C18:2 | | | 9,5 | 10,3 | 3,8 |
| - C18:3 | | | 3,9 | 4,3 | 1,6 |
| - C9,t11 CLA | | | 4,3 | 4,6 | 2,4 |
| - t10,c12 CLA | | | 4,3 | 4,5 | 2,1 |

| *Nasschemische Kennzahlen* | | | | | |
|---|---|---|---|---|---|
| Säurezahl | | 19 | 13 | 0,4 | 99 |
| Hydroxylzahl | | | | 2,5 | |
| Iodzahl | | | | 57 | |
| Verseifungszahl | | | | 251 | |
| Peroxidzahl | | | | 4,0 | |

**Tabelle 8**

| **Beschreibung des Reaktionsproduktes** | | | |
|---|---|---|---|
| **Farbzahlen** | **Sumpfprodukt** nach Destillation | **Produkt** gebleicht, raffiniert | **Rapsöl** |
| Gardner | 3,5 | 0,8 | 1,8 |
| Lovibond 5 ¼ | Ly 22,0; Lr 3,0 | Ly 6,4; Lr 0,9 | Ly 12,0; Lr 1,4 |
| Säurezahl | 0,4 | 0,1 | 0,4 |

Die Beispiele zeigen, dass über enzymatische Synthese und destillative Aufreinigung strukturierte Lipide hoher Qualität hergestellt werden können.

### Beispiel 6

### Herstellung strukturierter Lipide durch gekoppelte enzymatische Synthese und Umesterung mit destillativer Produktreinigung

In einen beheizbaren Doppelmantelreaktor wurden 450 g Caprylsäure, 180 g Caprinsäure, 135 g 80 Gew.-%ige CLA Fettsäure (Tonalin^{®} CLA 80), 142,5 g Glycerin sowie 900 g Rapsöl eingewogen. Zum Ansatz wurden 50 g immobilisierte *Candida antarctica B* Lipase gegeben und der Ansatz für 24 h bei 60 °C unter Rühren und Stickstoffbegasung bei einem Vakuum von 5 mbar inkubiert. Nach 5 h bzw. 24 h wurden jeweils Proben entnommen und gaschromatographisch sowie auf Säurezahl analysiert. Nach 24 h wurde das Enzym über Filtration vom Rohprodukt abgetrennt und zur weiteren Umesterung über eine mit immobilisierter *Thermomyces lanugenosus* Lipase gepackten Säule gepumpt. Die Säule war zu diesem Zweck mit 25 g immobilisierter Lipase gepackt und wurde bei Raumtemperatur mit einer Flussrate von 50 g/h betrieben. Das umgeesterte Produkt wurde gaschromatographisch sowie auf Säurezahl hin untersucht und anschließend bei 200 °C und einem Vakuum vom 0,4 mbar über eine Kurzwegdestillation aufgereinigt. Das Sumpfprodukt sowie das Destillat wurden gaschromatographisch und nasschemisch analysiert. Dabei werden die Verteilung der Glyceride sowie das Fettsäurespektrum analysiert, sowie die fettchemischen Kennzahlen des Sumpfproduktes bestimmt. Danach wurde das Sumpfprodukt mit 50 Gew.-% Natriumhydroxid (zweifache molare Menge bezogen auf freie Fettsäuren) bei 80 °C für 15 min raffiniert, 15 min bei 100 °C mit 2 Gew.-% Bleicherde Tonsil behandelt und mit 5 Gew.-% Wasser gewaschen. Die Wasserphase wurde anschließend abgetrennt und das Produkt im Vakuum getrocknet. Das fertige Produkt wurde zur Stabilisierung mit 500 ppm gemischten Tocopherolen (Covi-ox T-90) versetzt. Die Ergebnisse sind in Tabelle 9 zusammengefasst. Angegeben ist die Zusammensetzung in GC-Flächen-% sowie die Säurezahl. In Tabelle 10 sind die Säurezahlen sowie die Farbzahlen vor und nach der Raffination und Bleiche angegeben.

**Tabelle 9**

| **Zusammensetzung des Reaktionsproduktes** | | | | | |
|---|---|---|---|---|---|
| **Zusammensetzung** | **6a** | **6b** | **6c** | **6d** | **6e** |
| Produkt | 5h Synthese | 24 h Synthese | Umest. Säule | Destillation Sumpf | Destillation Destillat |
| *Glyceridverteilung [Flächen-%]* | | | | | |
| - FS (mittelkettig) | 14,8 | 4,2 | 3,5 | 0 | 20,5 |
| - FS (langkettig) | 5,6 | 3,4 | 3,0 | 0,3 | 24,4 |
| - MG (mittelkettig) | 0 | 0 | 0 | 0 | 0,3 |
| - MG (langkettig) | 0 | 0 | 0 | 0 | 0 |
| - DG (2x mittelkettig) | 15,1 | 1,7 | 1,8 | 0,2 | 12,4 |
| - DG (1x mittel-, 1x langkettig) | 5,3 | 0,4 | 1,0 | 0,5 | 1,0 |
| - DG (2x langkettig) | 0,8 | 0,9 | 0,7 | 0,8 | 0 |
| - TG (3x mittelkettig) | 16,9 | 21,0 | 18,7 | 16,0 | 38,4 |
| - TG (2x mittel-, 1x langkettig) | 7,9 | 34,8 | 37,9 | 42,9 | 3,0 |
| - TG (1x mittel-, 2x langkettig) | 4,0 | 25,1 | 27,4 | 32,2 | 0 |
| - TG (3x langkettig) | 29,6 | 8,5 | 6,0 | 7,1 | 0 |

| *Fettsäurespektrum* | | | | | |
|---|---|---|---|---|---|
| - C8 | | | 28,2 | 27,5 | 50,1 |
| - C10 | | | 13,4 | 13,8 | 16,5 |
| - C16:0 | | | 2,4 | 2,5 | 1,8 |
| - C18:0 | | | 1,2 | 1,2 | 0,8 |
| - C18:1 | | | 31,2 | 31,6 | 18,5 |
| - C18:2 | | | 10,0 | 10,3 | 4,6 |
| - C18:3 | | | 4,2 | 4,1 | 1,9 |
| - C9,t11 CLA | | | 4,8 | 4,5 | 3,1 |
| - t10,c12 CLA | | | 4,6 | 4,5 | 2,7 |

| *Nasschemische Kennzahlen* | | | | | |
|---|---|---|---|---|---|
| Säurezahl | 50 | 20 | 16 | 0,4 | 115 |
| Hydroxylzahl | | | | 5,4 | |
| Iodzahl | | | | 68 | |
| Verseifungszahl | | | | 255 | |
| Peroxidzahl | | | | 5,2 | |

**Tabelle 10**

| **Beschreibung des Reaktionsproduktes** | | | |
|---|---|---|---|
| **Farbzahlen** | **Sumpfprodukt** nach Destillation | **Produkt** gebleicht, raffiniert | **Rapsöl** |
| Gardner | 3,9 | 0,8 | 1,8 |
| Lovibond 5 ¼ | Ly 27,0; Lr 3,7 | Ly 6,3; Lr 0,8 | Ly 12,0; Lr 1,4 |
| Säurezahl | 0,4 | 0,4 | 0,4 |

Die Beispiele zeigen, dass durch enzymatische Synthese und Umesterung sind nach destillativer Reinigung qualitativ hochwertige strukturierte Lipide erhältlich sind.

### Beispiel 7

### Herstellung strukturierter Lipide durch enzymatische Umesterung

In drei Ansätzen wurden jeweils 17,5 g Caprylsäure, 7,5 g Caprinsäure, 5 g CLA-Triglycerid (Tonalin^{®} TG 80) sowie 20 g Rapsöl eingewogen. Zu den Ansätzen 1 und 2 wurden jeweils 2,5 g immobilisierte *Rhizomucor miehei* Lipase und zu dem Ansatz 3 2,5 g immobilisierte *Thermomyces lanugenosus* Lipase gegeben. Die Ansätze 1 und 3 wurden bei 45 °C und Ansatz 3 bei 60 °C unter Schütteln inkubiert. Nach 5 h, 24 h bzw. 48 h (von Ansatz 3 nur nach 5 h) wurden jeweils Proben entnommen und gaschromatographisch analysiert. Die Ergebnisse sind in Tabelle 11 zusammengefasst. Angegeben ist die Zusammensetzung in GC-Flächen-% sowie die Säurezahl.

**Tabelle 11**

| **Zusammensetzung des Reaktionsproduktes** | | | | | | | |
|---|---|---|---|---|---|---|---|
| **Zusammensetzung** | **7a** | **7b** | **7c** | **7d** | **7e** | **7f** | **7g** |
| Ansatz Nr. | 1 | 1 | 1 | 2 | 3 | 3 | 3 |
| Reaktionszeit [h] | 5 h | 24 h | 48 h | 5 h | 5 h | 24 h | 48 h |

| *Glyceridverteilung [Flächen-%]* | | | | | | | |
|---|---|---|---|---|---|---|---|
| - FS (mittelkettig) | 52,2 | 49,0 | 40,7 | 50,8 | 57,1 | 55,8 | 51,7 |
| - FS (langkettig) | 8,2 | 20,9 | 24,3 | 11,3 | 3,7 | 9,1 | 12,6 |
| - MG (mittelkettig) | 0 | 0 | 0 | 0 | 0 | 0 | 0 |
| - MG (langkettig) | 0 | 0 | 0 | 0 | 0 | 0 | 0 |
| - DG (2x mittelkettig) | 0,7 | 0,5 | 1,6 | 0,7 | 0,2 | 0 | 0,4 |
| -DG (1x mittel-, 1x langkettig) | 1,2 | 0,7 | 1,8 | 1,0 | 0,5 | 0,5 | 1,4 |
| - DG (2x langkettig) | 0,2 | 0 | 0,2 | 0,2 | 0,9 | 1,9 | 1,4 |
| -TG(3xmittelkettig) | 0 | 0 | 0,6 | 0 | 0 | 0 | 0,2 |
| - TG (2x mittel-, 1x langkettig) | 2,0 | 6,1 | 10,1 | 3,3 | 2,3 | 1,0 | 3,6 |
| - TG (1x mittel-, 2x langkettig) | 7,9 | 15,3 | 16,5 | 11,4 | 3,5 | 8,2 | 12,6 |
| - TG (3x langkettig) | 27,6 | 7,5 | 4,2 | 21,1 | 31,8 | 23,5 | 16,1 |
| Säurezahl | 182 | 184 | 184 | 188 | 188 | 190 | 190 |

Die Beispiele zeigen, dass die Umesterung mit beiden immobilisierten Lipasen sowohl bei 45°C als auch bei 60 °C erfolgreich durchgeführt werden kann. Bei Anwesenheit von hohen

### Beispiel 8

### Herstellung strukturierter Lipide durch enzymatische Umesterung mit destillativer Produktreinigung

In einen Kolben wurden 875 g Caprylsäure, 375 g Caprinsäure, 250 g CLA-Triglycerid (Tonalin^{®} TG 80) sowie 1000 g Rapsöl eingewogen. Der Ansatz wurde mit Stickstoff überschichtet, mit 150 g immobilisierte *Rhizomucor miehei* Lipase versetzt und für 48 h unter Schütteln inkubiert. Die ersten 24 h wurde bei einer Temperatur von 45 °C inkubiert, danach wurde die Temperatur auf 60 °C erhöht. Nach 5 h, 24 h bzw. 48 h wurden jeweils Proben entnommen und gaschromatographisch sowie auf Säurezahl analysiert. Nach 48 h wurde das Enzym über Filtration vom Rohprodukt entfernt. Das Rohprodukt wurde bei 200 °C und einem Vakuum vom 0,4 mbar zweimal über eine Kurzwegdestillation aufgereinigt und das so erhaltene Sumpfprodukt sowie das vereinigte Destillat gaschromatographisch und nasschemisch analysiert. Dabei wurden die Verteilung der Glyceride sowie das Fettsäurespektrum analysiert und die fettchemischen Kennzahlen des Sumpfproduktes bestimmt. Das Sumpfprodukt wurde mit 50 Gew.-% Natriumhydroxid (zweifache molare Menge bezogen auf freie Fettsäuren) bei 80 °C für 15 min raffiniert, 15 min bei 100 °C mit 2 % Bleicherde Tonsil behandelt und mit 5 Gew.-% Wasser gewaschen. Anschließend wurde die Wasserphase abgetrennt und der Rückstand im Vakuum getrocknet. Das fertige Produkt wird zur Stabilisierung mit 500 ppm gemischten Tocopherolen (Covi-ox T-90) versetzt. Die Säurezahlen sowie die Farbzahlen nach Lovibond und Gardner werden vor und nach der Raffination und Bleiche ermittelt. Die Ergebnisse sind in Tabelle 12 zusammengefasst. Angegeben ist die Zusammensetzung in GC-Flächen-% sowie die Säurezahl. In Tabelle 13 sind die Säurezahlen sowie die Farbzahlen nach Lovibond und Gardner vor und nach der Raffination und Bleiche angegeben.

**Tabelle 12**

| **Zusammensetzung des Reaktionsproduktes** | | | | | |
|---|---|---|---|---|---|
| **Zusammensetzung** | **8a** | **8b** | **8c** | **8d** | **8e** |
| Produkt | 5 h Synthese | 24 h Synthese | 48 h Synthese | Destillation Sumpf | Destillation Destillat |
| *Glyceridverteilung [Flächen-%]* | | | | | |
| - FS (mittelkettig) | 54,0 | 48,0 | 44,0 | 0,1 | 59,2 |
| - FS (langkettig) | 7,1 | 18,5 | 26,0 | 1,3 | 37,8 |
| - MG (mittelkettig) | 0 | 0 | 0 | 0 | 0,1 |
| - MG (langkettig) | 0 | 0 | 0 | 0 | 0,1 |
| - DG (2x mittelkettig) | 0,4 | 0,9 | 0,4 | 0,1 | 0,7 |
| - DG (1x mittel-, 1x langkettig) | 0,3 | 0,3 | 0,7 | 0,8 | 0 |
| - DG (2x langkettig) | 0,5 | 0,3 | 0,5 | 0,6 | 0 |
| - TG (3x mittelkettig) | 0 | 0,5 | 1,3 | 5,6 | 1,1 |
| - TG (2x mittel-, 1x langkettig) | 0,9 | 6,2 | 8,7 | 30,4 | 0,1 |
| - TG (1x mittel-, 2x langkettig) | 8,1 | 17,6 | 15,7 | 48,3 | 0 |
| - TG (3x langkettig) | 28,7 | 7,7 | 2,7 | 12,8 | 0 |

| *Fettsäurespektrum* | | | | | |
|---|---|---|---|---|---|
| - C8 | | | 35,3 | 16,9 | 41,9 |
| - C10 | | | 17,1 | 8,4 | 19,3 |
| - C16:0 | | | 2,2 | 2,4 | 2,0 |
| - C18:0 | | | 1,1 | 1,3 | 0,9 |
| - C18:1 | | | 25,4 | 38,6 | 21,7 |
| - C18:2 | | | 7,2 | 15,0 | 5,3 |
| - C18:3 | | | 3,2 | 6,3 | 2,1 |
| - C9,t11 CLA | | | 4,3 | 5,5 | 3,5 |
| - t10,c12 CLA | | | 4,1 | 5,6 | 3,3 |

| *Nasschemische Kennzahlen* | | | | | |
|---|---|---|---|---|---|
| Säurezahl | | | 185 | 3,1 | 290 |
| Hydroxylzahl | | | | 6,6 | |
| Iodzahl | | | | 62 | |
| Verseifungszahl | | | | 234 | |
| Peroxidzahl | | | | 4,1 | |

**Tabelle 13**

| **Beschreibung des Reaktionsproduktes** | | | |
|---|---|---|---|
| **Farbzahlen** | **Sumpfprodukt** nach Destillation | **Produkt** gebleicht, raffiniert | **Rapsöl** |
| Gardner | 7,1 | 3,1 | 1,8 |
| Lovibond 5 ¼ | Ly 107; Lr 15,2 | Ly 34,0; Lr 3,9 | Ly 12,0; Lr 1,4 |
| Säurezahl | 3,1 | 0,6 | 0,4 |

Die Beispiele zeigen, dass durch enzymatische Umesterung und destillative Reinigung strukturierte Lipide hoher Qualität hergestellt werden können.

### Beispiel 9

### Herstellung strukturierter Lipide mit verschiedenen pflanzlichen und tierischen Ölen

In fünf verschließbaren Kolben wurden jeweils 7,5 g MCT, 1,5 g CLA-Triglycerid (Tonalin^{®} TG 80), 0,5 g Lipozym^{®} TL IM und die nachfolgenden pflanzlichen und tierischen Öle gefüllt:
Ansatz 1 : 1,5 g Thunfischöl und 4,5 g Rapsöl
Ansatz 2 : 1,5 g Makrelenöl und 4,5 g Rapsöl
Ansatz 3 : 1,5 g Leinöl und 4,5 g Sojaöl
Ansatz 4 : 1,5 g Leinöl und 4,5 g Sonnenblumenöl
Ansatz 5 : 3,0 g Olivenöl und 3,0 g Rapsöl

Die Gefäße wurden verschlossen und für 24 h bei 45 °C unter Schütteln inkubiert. Nach beendeter Reaktion wurde eine Probe silyliert und gaschromatographisch auf die Glyceridverteilung untersucht. Es wurde der Gehalt an Triglyceridspecies bestimmt, wobei der Gehalt an Triglyceriden auf 100 normiert wurde. Zusätzlich wurde eine Probe methyliert und gaschromatographisch die Fettsäurezusammensetzung bestimmt. Die Ergebnisse sind in Tabelle 14 zusammengefasst. Angegeben ist die Zusammensetzung in GC-Flächen-%.

**Tabelle 14**

| **Zusammensetzung des Reaktionsproduktes** | | | | | |
|---|---|---|---|---|---|
| **Zusammensetzung** | **9a** | **9b** | **9c** | **9d** | **9e** |
| *Glyceridverteilung [Flächen-%]* | | | | | |
| - 3x mittelkettig | 35,3 | 36,4 | 33,6 | 32,5 | 32,1 |
| - 2x mittel-, 1x langkettig | 34,3 | 33,0 | 34,6 | 34,8 | 33,3 |
| - 1x mittel-, 2x langkettig | 18,7 | 18,0 | 18,4 | 20,5 | 20,4 |
| - 3x langkettig | 11,7 | 12,7 | 13,4 | 12,1 | 14,3 |

| *Fettsäurespektrum* | | | | | |
|---|---|---|---|---|---|
| - C8 | 38,2 | 37,4 | 37,0 | 36,5 | 36,5 |
| -C10 | 16,6 | 16,3 | 16,0 | 15,8 | 16,0 |
| - C16:0 | 3,4 | 3,3 | 4,2 | 2,6 | 2,8 |
| - C18:0 | 1,3 | 1,3 | 1,8 | 1,7 | 1,4 |
| - C18:1 | 20,6 | 20,8 | 10,5 | 12,3 | 28,7 |
| - C18:2 | 6,2 | 6,5 | 17,2 | 18,6 | 5,8 |
| - C18:3 | 2,2 | 2,3 | 6,0 | 4,7 | 1,6 |
| - C9,t11 CLA | 4,0 | 4,3 | 3,6 | 3,9 | 3,6 |
| - t10,c12 CLA | 3,8 | 4,1 | 3,6 | 3,9 | 3,5 |
| - C20:5 | 2,2 | 2,3 | 0 | 0 | 0 |
| - C22:6 | 1,5 | 1,4 | 0 | 0 | 0 |

Die Beispiele zeigen, dass die Herstellung von strukturierten Lipiden unabhängig von der Natur der eingesetzten Fetten und Öle mit Erfolg durchgeführt werden konnte.

### Beispiel 10

### Herstellung strukturierter Lipide vom Typ ABA mit verschiedenen pflanzlichen und tierischen Ölen

Die Beispiele 10a bis 10e wurden wiederholt, jedoch die Menge an MCT gegen ebenfalls 7,5 g Caprylsäure ausgetauscht. Die Ergebnisse sind in Tabelle 15 zusammengefasst. Angegeben ist die Zusammensetzung in GC-Flächen-%.

**Tabelle 15**

| **Zusammensetzung des Reaktionsproduktes** | | | | | |
|---|---|---|---|---|---|
| **Zusammensetzung** | **10a** | **10b** | **10c** | **10d** | **10e** |
| *Glyceridverteilung [Flächen-%]* | | | | | |
| - 3x mittelkettig | 2,0 | 1,5 | 1,8 | 1,8 | 1,7 |
| - 2x mittel-, 1x langkettig | 26,9 | 14,7 | 25,9 | 26,4 | 24,6 |
| - 1x mittel-, 2x langkettig | 39,2 | 33,6 | 38,3 | 38,0 | 37,2 |
| - 3x langkettig | 31,8 | 40,2 | 34,0 | 33,7 | 36,5 |

Auch diese Beispiele zeigen, dass die Herstellung von strukturierten Lipiden unabhängig von der Natur der eingesetzten Fetten und Öle mit Erfolg durchgeführt werden konnte.

### Beispiel 11

### Herstellung strukturierter Lipide mit verschiedenen Gehalten an mittelkettigen Fettsäuren

In vier verschließbare Kolben wurden MCT, CLA-Triglycerid (Tonalin^{®} TG 80) und Rapsöl in unterschiedlichen Mengen eingewogen. In Ansatz 1 wurden 5 g MCT, 1,5 g CLA-Triglycerid und 8,5 g Rapsöl, in Ansatz 2 in entsprechender Reihenfolge 7,5 g, 1,5 g und 6 g, in Ansatz 3 10 g, 1,5 g und 3,5 g und in Ansatz 4 7,5 g, 3 g und 4,5 g eingesetzt. Nach Zugabe von 0,5 g Lipozym^{®} TL IM wurden die Gefäße verschlossen und für 24 h bei 45 °C inkubiert. Nach beendeter Reaktion wurde eine Probe silyliert und gaschromatographisch auf die Glyceridverteilung untersucht. Es wurde der Gehalt an Triglyceridspecies bestimmt, wobei der Gehalt an Triglyceriden auf 100 normiert wurde. Zusätzlich wurde eine Probe methyliert und gaschromatographisch die Fettsäurezusammensetzung bestimmt. Die Ergebnisse sind in Tabelle 16 zusammengefasst. Angegeben ist die Zusammensetzung in GC-Flächen-%.

**Tabelle 16**

| **Zusammensetzung des Reaktionsproduktes** | | | | |
|---|---|---|---|---|
| **Zusammensetzung** | **11a** | **11b** | **11c** | **11d** |
| *Glyceridverteilung [Flächen-%]* | | | | |
| - 3x mittelkettig | 16,0 | 33,2 | 52,9 | 34,1 |
| - 2x mittel-, 1x langkettig | 30,8 | 34,3 | 30,3 | 28,4 |
| - 1x mittel-, 2x langkettig | 27,3 | 20,8 | 10,5 | 18,3 |
| - 3x langkettig | 25,8 | 11,7 | 6,2 | 19,2 |

| *Fettsäurespektrum* | | | | |
|---|---|---|---|---|
| - C8 | 23,6 | 36,2 | 49,5 | 35,2 |
| - C10 | 10,3 | 15,9 | 21,5 | 15,3 |
| - C16:0 | 3,1 | 2,1 | 1,2 | 1,7 |
| - C18:0 | 1,4 | 1,1 | 0,7 | 1,2 |
| - C18:1 | 36,7 | 25,6 | 14,0 | 21,3 |
| - C18:2 | 12.1 | 8,2 | 4,3 | 6,5 |
| - C18:3 | 4,8 | 3,1 | 1,6 | 2,6 |
| - C9,t11 CLA | 4,0 | 4,0 | 3,6 | 8,2 |
| - t10,c12 CLA | 4,0 | 3,9 | 3,5 | 8,0 |

Die Beispiele zeigen, dass die Herstellung der strukturierten Lipide unabhängig von den gewählten Einsatzverhältnisse erfolgreich durchgeführt werden kann.

### Beispiel 12

### Herstellung strukturierter Lipide vom ABA-Typ mit verschiedenen Gehalten an mittelkettigen Fettsäuren

Die Beispiele 50 bis 53 wurden wiederholt Menge an MCT gegen eine gewichtsgleiche Menge an Caprylsäure ersetzt. Nach beendeter Reaktion wurde eine Probe silyliert und gaschromatographisch auf die Glyceridverteilung untersucht. Es wurde der Gehalt an Triglyceridspecies bestimmt, wobei der Gehalt an Triglyceriden auf 100 normiert wurde. Zusätzlich wurde eine Probe methyliert und gaschromatographisch die Fettsäurezusammensetzung bestimmt. Die Ergebnisse sind in Tabelle 17 zusammengefasst. Angegeben ist die Zusammensetzung in GC-Flächen-%.

**Tabelle 17**

| **Zusammensetzung des Reaktionsproduktes** | | | | |
|---|---|---|---|---|
| **Zusammensetzung** | **12a** | **12b** | **12c** | **12d** |
| *Glyceridverteilung [Flächen-%]* | | | | |
| - 3x mittelkettig | 1,5 | 1,3 | 0,7 | 2,1 |
| - 2x mittel-, 1x langkettig | 31,3 | 23,6 | 16,2 | 27,4 |
| - 1x mittel-, 2x langkettig | 51,2 | 35,0 | 18,8 | 37,9 |
| - 3x langkettig | 16,0 | 40,1 | 64,3 | 32,6 |

Auch diese Beispiele zeigen, dass die Herstellung der strukturierten Lipide unabhängig von den gewählten Einsatzverhältnisse erfolgreich durchgeführt werden kann.

### Beispiel 13

### Vergleich der regioselektiven Fettsäurezusammensetzung der strukturierten Lipide

Die destillierten Lipide aus den Beispielen 2, 3, 5, 6 und 8 wurden auf ihre regioselektive Fettsäurestruktur untersucht. Dazu wurde eine regioselektive enzymatische Alkoholyse mit immobilisierter *Thermomyces lanugenosus* Lipase bei 20 °C durchgeführt. Es wurden jeweils 0,5 g strukturiertes Lipid mit 1,5 g Ethanol gemischt und mit Lipase unter Rühren inkubiert. Die regioselektiv gespaltenen Produkte wurden silyliert und einer gaschromatographischen Bestimmung unterzogen. Die Peakflächen der gebildeten Ethylester wurden ausgewertet und die Gesamtsumme der Ethylester = 100 gesetzt. Zur Bestimmung der Fettsäurezusammensetzung an der 2-Position wurde die Differenz der Fettsäure-Gesamtverteilung und der Verteilung an der 1- und 3-Position gebildet. Die Gesamtverteilung wurde über Methylierung der strukturierten Lipide und gaschromatographische Analyse bestimmt. Zur Bestimmung der Verteilung an der 2-Position wurde der Massenunterschied der Methylester sowie der analysierten Ethylester rechnerisch extrapoliert. Die Gesamtheit der Fettsäuren an den Positionen 1,3 und 2 wurde jeweils auf 100 normiert. Die Ergebnisse sind in Tabelle 18 zusammengefasst. Angegeben sind jeweils GC-Flächen-%.

**Tabelle 18**

| **Zusammensetzung des Reaktionsproduktes** | | | |
|---|---|---|---|
| **Fettsaure** | **Gesamt** | **1,3-Position** | **2-Position** |
| *Muster aus Beispiel 2* | | | |
| - C8 | 29,7 | 31,7 | 25,7 |
| - C10 | 13,9 | 13,8 | 14,0 |
| - C16:0 | 2,4 | 2,5 | 2,2 |
| - C18:0 | 1,2 | 1,2 | 1,3 |
| - C18:1 | 29,9 | 29,2 | 31,3 |
| - C18:2 | 9,4 | 8,0 | 12,3 |
| - C18:3 | 3,9 | 3,5 | 4,8 |
| - C9,t11 CLA | 4,8 | 5,1 | 4,3 |
| - t10,c12 CLA | 4,7 | 5,1 | 4,0 |

| *Muster aus Beispiel 3* | | | |
|---|---|---|---|
| - C8 | 20,5 | 21,0 | 19,5 |
| - C10 | 9,2 | 9,0 | 9,7 |
| - C16:0 | 3,0 | 3,4 | 2,4 |
| - C18:0 | 1,3 | 1,5 | 1,0 |
| - C18:1 | 38,5 | 39,6 | 36,1 |
| - C18:2 | 12,6 | 11,1 | 15,6 |
| - C18:3 | 5,5 | 4,7 | 7,1 |
| - C9,t11 CLA | 4,7 | 4,9 | 4,4 |
| - t10,c12 CLA | 4,6 | 4,9 | 4,1 |

| *Muster aus Beispiel 5* | | | |
|---|---|---|---|
| - C8 | 27,2 | 29,7 | 22,1 |
| - C10 | 13,8 | 13,6 | 14,1 |
| - C16:0 | 2,5 | 2,6 | 2,3 |
| - C18:0 | 1,2 | 1,3 | 1,1 |
| - C18:1 | 31,7 | 30,5 | 34,0 |
| - C18:2 | 10,3 | 8,4 | 14,0 |
| - C18:3 | 4,3 | 3,5 | 5,8 |
| - C9,t11 CLA | 4,6 | 5,2 | 3,5 |
| - t10,c12 CLA | 4,5 | 5,2 | 3,1 |

| *Muster aus Beispiel 6* | | | |
|---|---|---|---|
| - C8 | 27,5 | 27,9 | 26,7 |
| - C10 | 13,8 | 13,0 | 15,4 |
| - C16:0 | 2,5 | 2,7 | 2,1 |
| - C18:0 | 1,2 | 1,4 | 1,0 |
| - C18:1 | 31,6 | 31,9 | 30,9 |
| - C18:2 | 10,3 | 8,9 | 13,1 |
| - C18:3 | 4,1 | 3,9 | 4,5 |
| - C9,t11 CLA | 4,5 | 5,2 | 3,1 |
| - t10,c12 CLA | 4,5 | 5,2 | 3,1 |

| *Muster aus Beispiel 8* | | | |
|---|---|---|---|
| - C8 | 16,9 | 23,9 | 2,9 |
| - C10 | 8,4 | 10,9 | 3,5 |
| - C16:0 | 2,4 | 2,8 | 1,5 |
| - C18:0 | 1,3 | 1,4 | 1,2 |
| - C18:1 | 38,6 | 35,0 | 45,7 |
| - C18:2 | 15,0 | 10,2 | 24,5 |
| - C18:3 | 6,3 | 4,4 | 10,1 |
| - C9,t11 CLA | 5,5 | 5,6 | 5,3 |
| - t10,c12 CLA | 5,6 | 5,8 | 5,3 |

Die Beispiele zeigen, dass die Regiostruktur des eingesetzten Rapsöls artiell erhalten bleibt, mehrfach ungesättigte Fettsäuren sind bevorzugt in der 2-Position zu finden. Das Muster aus Beispiel 8 besitzt eine ABA-Typ Struktur mit mittelkettigen Fettsäuren in der 1,3-Position und langkettigen Fettsäuren in der 2-Position.

### Beispiel 14

### Vergleich des Gehalts an trans-Fettsäuren der strukturierten Lipide

Die destillierten Lipide aus den Beispielen 2, 3, 5, 6 und 8 wurden auf ihren Gehalt an trans-Fettsäuren im Vergleich zum eingesetzten Rapsöl untersucht. Die Untersuchung wurde gaschromatographisch nach Methylierung der Lipide durchgeführt. Konjugierte trans-Verbindungen (CLA) wurden bei der Auswertung nicht berücksichtigt. Die Ergebnisse sind in Tabelle 18 zusammengefasst. Angegeben sind jeweils GC-Flächen-%.

**Tabelle 19**

| **Zusammensetzung des Reaktionsproduktes (Forts.)** | | | | | | |
|---|---|---|---|---|---|---|
| **Fettsäure** | **Bsp. 2** | **Bsp. 3** | **Bsp. 5** | **Bsp. 6** | **Bsp. 8** | **Rapsöl** |
| 18:1 trans | <0,1 | <0,1 | <0,1 | <0,1 | <0,1 | <0,1 |
| 18:2 trans | <0,3 | <0,3 | <0,3 | <0,3 | <0,3 | 0,3 |
| 18:3 trans | <0,5 | <0,8 | <0,7 | <0,7 | <1,0 | <1,3 |
| Gesamt | 0,6 | 1,0 | 0,8 | 0,8 | 1,1 | 1,5 |

Die Beispiele zeigen, dass während der enzymatischen Umsetzung und der Aufarbeitung keine zusätzlichen unerwünschten trans-Fettsäuren entstehen. Die in den Produkten analysierten trans-Gehalte stammen aus dem eingesetzten Ausgangsprodukt Rapsöl. Die Muster enthalten somit nur die ernährungsphysiologisch positiven konjugierten Linolsäuren.

### Beispiel 15

### Vergleich der Oxidationsstabilitäten der strukturierten Lipide

Die destillierten Lipide aus den Beispielen 2, 3, 5, 6 und 8 wurden auf ihre Oxidationsstabilität untersucht. Dazu wurden Untersuchungen in einem Metrohm Rancimaten bei 100 °C durchgeführt. Die destillierten Lipide wurden sowohl mit als auch ohne Antioxidants (Covi-ox T-90) stabilisiert und im Vergleich zu MCT, CLA-Triglycerid und Rapsöl untersucht. Rapsöl enthält natürliche Antioxidantien, die die Oxidationsstabilität positiv beeinflussen. Die Ergebnisse sind in Tabelle 20 zusammengefasst.

**Tabelle 20**

| **Oxidationsstabilität [h]** | | |
|---|---|---|
| **Muster aus** | **ohne Antioxidants** | **mit Antioxidants** |
| Beispiel 3 | 3 | 11 |
| Beispiel 4 | 3 | 13 |
| Beispiel 5 | 6 | 16 |
| Beispiel 6 | 6 | 9 |
| Beispiel 8 | 2 | 4 |
| Rapsöl | 12 | |
| MCT | > 100 | |
| CLA Triglycerid | | 6 |

Die Beispiele zeigen, dass die mit dem Antioxidants behandelten strukturierten Lipide eine vergleichbare Stabilität zum Rapsöl auf und eine bessere Stabilität als das CLA-Triglycerid aufweisen.

### Beispiel 16

### Vergleich der physikalischen Eigenschaften Viskosität und Trübungspunkt der strukturierten Lipide

Die destillierten Lipide aus den Beispielen 2, 3, 5, 6 und 8 wurden auf ihre physikalischen Eigenschaften untersucht. Dazu wurde der Cloud Point nach ASTM D2500 mit einem ATPEM V4701 analysiert. Die Viskositäten wurden rheometrisch bei unterschiedlichen Temperaturen aufgenommen. Alle Messungen wurden vergleichend mit Rapsöl, MCT und CLA-Triglycerid durchgeführt. Die Ergebnisse sind in Tabelle 21 zusammengefasst.

**Tabelle 21**

| **Trübungspunkt und Viskosität** | | | | | |
|---|---|---|---|---|---|
| **Muster aus** | **Trübungspunkt** [**°C**] | **Viskosität [mm²/s] bei** | | | |
| | | **10 °C** | **30 °C** | **50 °C** | **70 °C** |
| Beispiel 2 | -20 | 84 | 35 | 17 | 10 |
| Beispiel 3 | -26 | 99 | 49 | 20 | 12 |
| Beispiel 5 | -17 | 88 | 36 | 18 | 11 |
| Beispiel 6 | -17 | 90 | 37 | 18 | 11 |
| Beispiel 8 | - 30 | | | | |
| Rapsöl | -33 | 130 | 53 | 26 | 15 |
| MCT | -59 | 47 | 20 | 10 | 6 |
| CLA-TG | | 281 | 96 | 42 | 22 |

Wie die Beispiele zeigen, liegt der Trübungspunkt der strukturierten Lipide im Bereich von Rapsöl und anderen Speiseölen. Die Viskosität der strukturierten Lipide liegt ebenfalls im Bereich von Rapsöl.

### Beispiel 17

### Einarbeitung der strukturierten Lipide in Milch

Die Muster aus Beispiel 3 und 5 wurden in unterschiedlichen Konzentrationen in Milch eingearbeitet. Zu Milch mit einem Fettanteil von 1,5 Gew.-% wurden jeweils 1 Gew.-% bzw. 4 Gew.-% der Muster gegeben und das Gemisch wurde für 30 Sekunden mit einem Ultra-Turrax bei 9500 U/min homogenisiert. Es wurde jeweils eine weiße stabile und homogene Milch hergestellt.

### Beispiel 18

### Einarbeitung der strukturierten Lipide in Mayonnaise

Die Muster aus Beispiel 3 und 5 wurden mit einem Anteil von 6 Gew.-% in Mayonnaise eingearbeitet. Dazu wurden 17,8 Gew.-% Wasser mit 2 Gew.-% Lamegin^{®} ZE 609 Pastille bei 65 °C zu einer Paste gerührt. Daraufhin wurden 19 Gew.-% Sonnenblumenöl und jeweils 6 Gew.-% der strukturierten Lipide auf 40 °C erwärmt und zu der Emulgator-Wasser Paste hinzu gegeben. Danach wurde die Wasserphase bestehend aus 4 Gew.-% Essig (5 Vol.-%ig), 50 Gew.-% Wasser, 0,5 Gew.-% Prinza^{®} 452 und 0,7 Gew.-% Kochsalz, auf 65 °C erwärmt und unter Rühren zu der Ölpaste hinzu gegeben. Unter Rühren wurde die Mayonnaise auf Raumtemperatur abgekühlt. Es wurde jeweils eine weiße stabile Mayonnaise Formulierung hergestellt.

### Beispiel 19

### Rauchentwicklung der strukturierten Lipide beim Erhitzen

Mit den Mustern aus Beispiel 3 und 5 wurde eine Rauchpunktsbestimmung im Vergleich zu Mischungen aus Rapsöl und MCT (durchgeführt. Die angegebenen Werte sind Mittelwerte aus 2 Messungen. Die Ergebnisse sind in Tabelle 22 zusammengefasst.

**Tabelle 22**

| **Rauchpunkte** | | |
|---|---|---|
| **Muster aus** | **Mittelkettige Fettsäuren** [**Gew.-%**] | **Rauchpunkt [°C]** |
| Beispiel 3 | 29,7 | 169 |
| Beispiel 5 | 40,0 | 171 |
| 100 % Rapsöl | 0 | 180 |
| 90 % Rapsöl + 10 % MCT | 10 | 170 |
| 80 % Rapsöl + 20 % MCT | 40 | 163 |
| 60 % Rapsöl + 40 % MCT | 60 | 160 |
| 20 % Rapsöl + 80 % MCT | 80 | 156 |
| 100 % MCT | 100 | 152 |

Die Beispiele zeigen, dass die strukturierten Lipide ein deutlich besseres Rauchverhalten als Mischungen mit den entsprechenden Konzentrationen an MCT und Rapsöl aufweisen.

### Beispiel 20

### Herstellung strukturierter Lipide durch enzymatische Umesterung mit destillativer Produktreinigung

In einen Doppelmantelreaktor wurden 500 g MCT (Delios^{®} V), 100 g CLA-Triglycerid (Tonalin TG 80) sowie 400 g Rapsöl (Vollraffinat) und 0,5 g Covi-ox^{®} T-90 vorgelegt. Der Ansatz wurde mit 100 g immobilisierter *Thermomyces lanugenosus* Lipase versetzt und das Enzym wurde für 3 h unter Rühren bei 45 °C und einem Vakuum von 20 mbar getrocknet. Anschließend wurde das immobilisierte Enzym abfiltriert und wieder in den Reaktor zurückgeführt. Dazu wurden 1000 g MCT (Delios^{®} V), 200 g CLA-Triglycerid (Tonalin^{®} TG 80) sowie 800 g Rapsöl (Vollraffinat) und 1,0 g Covi-ox^{®} T-90 gegeben und der Ansatz wurde mit Stickstoff überschichtet. Der Ansatz wurde bei 45 °C unter Rühren für 24 h inkubiert. Nach 24 h wurde das Enzym über Filtration vom Rohprodukt entfernt. Das Rohprodukt wurde bei 205 °C und einem Vakuum vom 0,3 mbar über eine Kurzwegdestillation aufgereinigt. Das Sumpfprodukt wurde gaschromatographisch und nasschemisch analysiert. Dabei wurden die Verteilung der Glyceride sowie das Fettsäurespektrum analysiert, sowie die fettchemischen Kennzahlen bestimmt. Die Ergebnisse sind in Tabelle 23 zusammengefasst. Zusätzlich wurde vom Produkt eine Desodorierung mit Trägergas im Gegenstrom zum sensorischen Vergleich durchgeführt.

**Tabelle 23**

| **Zusammensetzung des Reaktionsproduktes** | |
|---|---|
| **Zusammmensetzung** | **Produkt** |
| *Glyceridverteilung [Flächen-%]* | |
| Freie Säure gesamt | 0,3 |
| Monoglyceride gesamt | <0,1 |
| Diglyceride gesamt | 1,0 |
| - TG (3x mittelkettig) | 16,1 |
| - TG (2x mittel-, 1x langkettig) | 47,7 |
| - TG (1x mittel-, 2x langkettig) | 3 0,4 |
| - TG (3x langkettig) | 4,5 |

| *Fettsäurespektrum [Flächen-%]* | |
|---|---|
| - C8 | 24,1 |
| - C10 | 19,9 |
| - C16:0 | 2,2 |
| -C18:0 | 1,1 |
| - C18:1 | 28,2 |
| -C18:2 | 10,0 |
| -C18:3 | 4,9 |
| - c9,t1 CLA | 4,9 |
| - t10,c12 CLA | 4,9 |

| *Nasschemische Kennzahlen* | |
|---|---|
| Säurezahl | 0,1 |
| Hydroxylzahl | 4,3 |
| Iodzahl | 67 |
| Verseifungszahl | 256 |
| Peroxidzahl | 2,3 |

Das Beispiel zeigt, dass über enzymatische Umesterung unter Inertgasathmosphäre mit vorgetrocknetem Enzym und destillative Reinigung strukturierte Lipide hoher Qualität hergestellt werden können.

### Beispiel 21

### Rauchentwicklung und Oxidationsstabilität des strukturierten Lipids aus Beispiel 20

Mit dem Muster aus Beispiel 20 wurde eine Rauchpunktsbestimmung im Vergleich zu den eingesetzten Rohmaterialien sowie eine Oxidationsstabilität bei 100 °C im Vergleich zum eingesetzten Rapsöl und Tonalin^{®} TG 80 bestimmt. Die Ergebnisse sind in Tabelle 24 zusammengefasst.

**Tabelle 24**

| **Oxidationsstabilität und Rauchpunkt** | | |
|---|---|---|
| **Muster aus** | **Oxidationsstabilität [h]** | **Rauchpunkt [°C]** |
| Beispiel 20 | 20 | 187 |
| Tonalin^{®} TG 80 | 6 | 185 |
| Delios^{®} V | | 149 |
| Rapsöl Vollraffinat | 8 | 191 |

Die Beispiele zeigen, dass das strukturierte Lipid eine deutlich bessere Oxidationsstabilität aufweist als die Edukte Tonalin^{®} TG 80 und Rapsöl und dass das Rauchverhalten vergleichbar zu den rein langkettigen Triglyceriden ist und deutlich besser als das mittelkettige Delios^{®} V. Vergleich zu den Mischungen mit den entsprechenden Konzentrationen an MCT und Rapsöl aus Beispiel 19 zeigen ein um etwa 25 °C verbessertes Rauchverhalten des strukturierten Lipids.

### Beispiel 22

### Vergleich der sensorischen Eigenschaften gegen Rapsöl

Die Muster aus Beispiel 20 (desodoriert und nicht desodoriert) wurden gegen das zur Synthese eingesetzte Rapsöl sensorisch auf Geruch und Geschmack untersucht. Zur Bewertung wurden von einem Panel von 3 geschulten Testpersonen die Parameter ranziger Geruch; Fremdgeruch, ranziger Geschmack, Fehlgeschmack und Bitterkeit analysiert. Die strukturierten Lipide schnitten in den Kategorien ranziger Geruch, ranziger Geschmack und Fehlgeschmack deutlich besser ab als Rapsöl. In den Kategorien Fremdgeruch und Bitterkeit wurden ähnliche Testergebnisse für die strukturierten Lipide und das Rapsöl erhalten mit einer leicht besseren Beurteilung der strukturierten Lipide. Das desodorierte strukturierte Lipid erzielte dabei eine leicht bessere Beurteilung als das nicht desodorierte Produkt.

### Beispiel 23

### Vergleich der sensorischen Eigenschaften gegen Rapsöl in Milch

Die Muster aus Beispiel 20 (desodoriert und nicht desodoriert) wurden gegen das zur Synthese eingesetzte Rapsöl sensorisch auf Geruch und Geschmack eingearbeitet in Milch untersucht. Zur Bewertung wurden von einem Panel von 3 geschulten Testpersonen die Parameter ranziger Geruch; Fremdgeruch, ranziger Geschmack, Fehlgeschmack, fischige Geschmacksnote und Bitterkeit analysiert. Dazu wurden die Muster und Rapsöl in unterschiedlichen Konzentrationen in Milch eingearbeitet. Zu Milch mit einem Fettanteil von 1,5 Gew.-% wurden jeweils 1 Gew.-%, 4 Gew.-% bzw. 8 Gew.-% der Muster gegeben und das Gemisch wurde für 30 Sekunden mit einem Ultra-Turrax bei 9500 U/min homogenisiert. Es wurde jeweils eine weiße stabile und homogene Milch hergestellt. Die in der Milch eingearbeiteten strukturierten Lipide schnitten in den Kategorien Fremdgeruch, Fehlgeschmack und fischige Geschmacksnote deutlich besser ab als Rapsöl. In den anderen Kategorien wurden ähnliche Testergebnisse für die strukturierten Lipide und das Rapsöl erhalten mit einer leicht besseren Beurteilung der strukturierten Lipide. Das desodorierte strukturierte Lipid erzielte dabei eine leicht bessere Beurteilung als das nicht desodorierte Produkt.

### Beispiel 24

### Vergleich der sensorischen Eigenschaften gegen Rapsöl in Mayonnaise

Die Muster aus Beispiel 20 (desodoriert und nicht desodoriert) wurden gegen das zur Synthese eingesetzte Rapsöl sensorisch auf Geruch und Geschmack eingearbeitet in Mayonnaise untersucht. Zur Bewertung wurden von einem Panel von 3 geschulten Testpersonen die Parameter ranziger Geruch; Fremdgeruch, ranziger Geschmack, Fehlgeschmack, fischige Geschmacksnote und Bitterkeit analysiert. Dazu wurden die Muster und Rapsöl mit einem Anteil von 6 Gew.-% in Mayonnaise eingearbeitet. Dazu wurden 17,8 Gew.-% Wasser mit 2 Gew.-% Lamegin^{®} ZE 609 Pastille bei 65 °C zu einer Paste gerührt. Daraufhin wurden 19 Gew.-% Sonnenblumenöl und jeweils 6 Gew.-% der strukturierten Lipide auf 40 °C erwärmt und zu der Emulgator-Wasser Paste hinzu gegeben. Danach wurde die Wasserphase bestehend aus 4 Gew.-% Essig (5 Vol.-%ig), 50 Gew.-% Wasser, 0,5 Gew.-% Prinza^{®} 452 und 0,7 Gew.-% Kochsalz, auf 65 °C erwärmt und unter Rühren zu der Ölpaste hinzu gegeben. Unter Rühren wurde die Mayonnaise auf Raumtemperatur abgekühlt. Es wurde jeweils eine weiße stabile Mayonnaise Formulierung hergestellt. Die in der Mayonnaise eingearbeiteten strukturierten Lipide schnitten in allen Kategorien vergleichbar zum eingesetzten Rapsöl ab.

### Beispiel 25

### Vergleich der Abbaugeschwindigkeit in simulierter Darmflüssigkeit

Die Muster aus Beispiel 2 und 5 wurden gegen eine Mischung aus Triglyceriden in simulierter Darmflüssigkeit auf ihre Hydrolysierbarkeit und die Geschwindigkeit der CLA-Freisetzung hin analysiert.Dazu wurde eine simulierte Darmflüssigkeit hergestellt. Lösung A: Zu 1,36 g Kaliumhydrogenphosphat wurden 38 ml 0,2 M NaOH und 70 ml Wasser dest. gegeben. Lösung B: 0,5 g Pankreatin werden mit Wasser zu einer klumpenfreien Paste verrührt. Wasser wird dabei bis zu einer Gesamtmenge von 30 ml unter Mischen zugegeben. Lösung C: Lösung A und Lösung B werden zusammengegeben. 50 mg Taurocholsäure werden unter Rühren zugegeben und der pH-Wert wird mit 0,2 M NaOH auf 7,5 eingestellt. Anschließend wird die Lösung auf 180 ml mit Wasser dest. Aufgefüllt.

Jeweils 2 g Muster der strukturierten Lipide aus Beispiel 2 und 5 sowie 2 g einer Lipidmischung bestehend aus 0,2 g CLA-Triglycerid (Tonalin TG 80), 0,8 g MCT und 1 g Rapsöl als Doppelbestimmung werden in 40 ml vorgewärmte Lösung C gegeben und bei 37 °C unter Schütteln inkubiert. Nach 1 h und 3 h werden jeweils 2 ml Probe entnommen und mit 0,1 ml 1 M HCl und 0,4 ml Oktanol für 10 min unter Schütteln extrahiert. Die lipidhaltige Oktanolphase wird abgenommen. 200 µl der Oktanolphase werden für 30 min bei 80 °C mit 800 µl BSTFA /MSTFA syliliert und gaschromatographisch analysiert. Die freigesetzten Fettsäuren werden anhand ihrer Fläche bezogen auf den Gehalt aller Lipidbestandteile (Triglyceride, Partialglyceride und Fettsäuren) ausgewertet. Die Ergebnisse sind in Tabelle 25 zusammengefasst. Die Werte für die Vergleichs-Lipidmischung sind Mittelwerte der Doppelbestimmung.

**Tabelle 25**

| **Fettsäurefreisetzung bei der Hydrolyse in simulierter Darmflüssigkeit** | | |
|---|---|---|
| **Muster aus Beispiel 2** | **Fettsäuregehalt nach 1 h** | **Fettsäuregehalt nach 3 h** |
| Mittelkettige FS | 4,9% | 14,1% |
| Langkettige FS | 9,2 % | 18,5 % |
| CLA | 1,2% | 2,6% |
| Summe | 15,3% | 35,2% |

| **Muster aus Beispiel 5.** | **Fettsäuregehalt nach 1 h** | **Fettsäuregehalt nach 3 h** |
|---|---|---|
| Mittelkettige FS | 4,0% | 13,2% |
| Langkettige FS | 6,7% | 12,5% |
| CLA | 1,2% | 2,5% |
| Summe | 11,9% | 28,2% |

| **Lipidmischung aus 10% CLA-TG, 40 % MCT und 50 % Rapsöl** | **Fettsäuregehalt nach 1 h** | **Fettsäuregehalt nach 3 h** |
|---|---|---|
| Mittelkettige FS | 2,6% | 15,0% |
| Langkettige FS | 2,0 % | 6,2 % |
| CLA | 0,4% | 1,2% |
| Summe | 5,0 % | 22,4 % |

Die Beispiele zeigen, dass die strukturierten Lipide in Summe wesentlich schneller hydrolysiert werden als eine Vergleichsmischung nicht struktuierter Lipide. Insbesondere die langkettigen Fettsäuren werden deutlich schneller freigesetzt als aus der Vergleichsmischung. Dies bedeutet, dass eine bessere Verfügbarkeit der essentiellen Fettsäuren gewährleistet ist und das eine schnellere Wirkung der CLA erfolgen kann.

## Patentansprüche

1. Gemisch enthaltend Lipide der Formel (I), 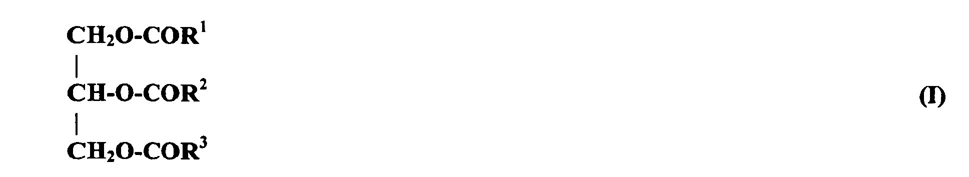 **dadurch gekennzeichnet, dass** R¹CO, R²CO und R³CO unabhängig voneinander ausgewählt sind aus der Gruppe bestehend aus
(v) einem linearen gesättigten Acylrest mit 6 bis 12 Kohlenstoffatomen,
(vi) dem Acylrest der konjugierten Linolsäure (CLA)
(vii) dem Acylrest einer omega-3 Fettsäure (OF) und
(viii) dem Acylrest einer mega-6 Fettsäure (OF),
mit der Maßgabe, dass die Menge an CLA-Acylresten in dem Gemisch 3 bis 50 Mol-% und die Menge an OF-Acylresten in dem Gemisch 5 bis 25 Mol-% - jeweils bezogen auf die Menge aller Acylreste in dem Gemisch - ausmacht.

2. Verfahren zur Herstellung des Gemisches nach Anspruch 1, **dadurch gekennzeichnet, dass** man
(a) so genannte "medium chain triglycerides" (MCT) der Formel (II), 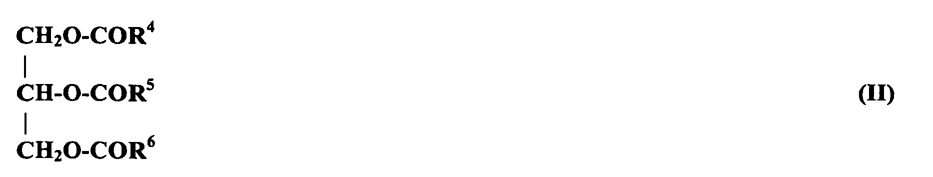 wobei R⁴CO, R⁵CO und R⁶CO unabhängig voneinander jeweils für einen linearen gesättigten Acylrest mit 6 bis 12 Kohlenstoffatomen stehen, und
(b) konjugierte Linolsäure (CLA) und/oder omega-3 Fettsäuren (OF) und/oder omega-6 Fettsäuren (OF) und/oder Triglyceride auf Basis konjugierter Linolsäure (TG-CLA) und/oder Triglyceride auf Basis von omega-3 oder von omega-6 Fettsäuren (TG-OF) einer enzymatischen Umesterung unterwirft.

3. Verfahren zur Herstellung des Gemisches nach Anspruch 1, **dadurch gekennzeichnet, dass** man
(a) Gemische von Fettsäuren oder deren Estern der Formel **(III),**
**R⁷COOR⁸** **(III)**
in der R⁷CO für einen linearen gesättigten Acylrest mit 6 bis 12 Kohlenstoffatomen und R⁸ für Wasserstoff oder einen Alkylrest mit 1 bis 4 Kohlenstoffatomen steht, und
(b) konjugierter Linolsäure (CLA) und/oder omega-3 und/oder omega-6 Fettsäuren einer enzymatischen Ver- bzw. Umesterung unterwirft.

4. Verwendung des Gemisches nach Anspruch 1 als Nahrungsmittel, Nahrungsergänzungsmittel, pharmazeutische Präparation oder Tierfutter.

5. Verwendung nach Anspruch 4, **dadurch gekennzeichnet, dass** die Nahrungsmittel Speise- bzw. Diätspeiseöle, Brat- und Frittieröle und der gleichen darstellen.

6. Verwendung nach Anspruch 4, **dadurch gekennzeichnet, dass** das Gemisch Bestandteil von Dressings, Mayonnaisen, Margarine, Milchprodukten, Säften, Back- und Süsswaren und der gleichen darstellt.

7. Verwendung nach Anspruch 4, **dadurch gekennzeichnet, dass** man das Gemisch zur Umesterung mit gehärteten Fetten und zur Erzeugung von Lipiden mit einstellbarem Solid Fat Content, Schmelz- und Kristallisationsverhalten einsetzt.

8. Verwendung nach Anspruch 4, **dadurch gekennzeichnet, dass** man das Gemisch zur parenteralen Ernährung einsetzt.

9. Verwendung nach einem der Ansprüche 4 bis 8, **dadurch gekennzeichnet, dass** man das Gemisch in Form von Emulsionen mit Phospholipiden oder Kapseln einsetzt.

10. Verwendung des Gemisches nach Anspruch 1 zur Herstellung eines Medikamentes zur Behandlung von Erkrankungen des Fettstoffwechsels.

## Claims

1. A mixture comprising lipids of the formula (I), 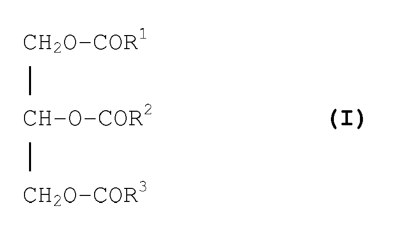 wherein R¹CO, R²CO and R³CO independently of one another are selected from the group consisting of
(v) a linear saturated acyl radical having 6 to 12 carbon atoms,
(vi) the acyl radical of conjugated linoleic acid (CLA),
(vii) the acyl radical of an omega-3 fatty acid (OF) and
(viii) the acyl radical of an omega-6 fatty acid (OF),
with the proviso that the amount of CLA-acyl radicals in the mixture makes up 3 to 50 mol% and the amount of OF-acyl radicals in the mixture makes up 5 to 25 mol% - in each case based on the amount of all acyl radicals in the mixture.

2. A method for producing the mixture according to claim 1, wherein
(a) what are termed "medium chain triglycerides" (MCT) of the formula (II), 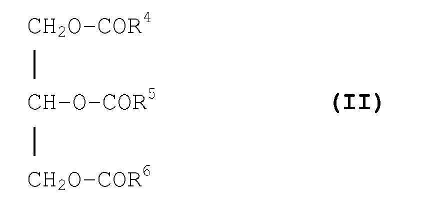 in which R⁴CO, R⁵CO and R⁶CO independently of one another are each a linear saturated acyl radical having 6 to 12 carbon atoms, and
(b) conjugated linoleic acid (CLA) and/or omega-3 fatty acids (OF) and/or omega-6 fatty acids (OF) and/or triglycerides based on conjugated linoleic acid (TG-CLA) and/or triglycerides based on omega-3 or omega-6 fatty acids (TG-OF) are subjected to an enzymatic transesterification.

3. A method for producing the mixture according to claim 1, wherein
(a) mixtures of fatty acids or esters thereof of the formula **(III)**,
R⁷COOR⁸ **(III)**
in which R⁷CO is a linear saturated acyl radical having 6 to 12 carbon atoms and R⁸ is hydrogen or an alkyl radical having 1 to 4 carbon atoms, and
(b) conjugated linoleic acid (CLA) and/or omega-3 and/or omega-6 fatty acids are subjected to an enzymatic esterification or transesterification.

4. The use of the mixture according to claim 1 as foodstuff, foodstuff supplement, pharmaceutical preparation or animal feed.

5. The use according to claim 4, wherein the foodstuffs are edible oils or dietetic edible oils, frying oils and deep-frying oils and the like.

6. The use according to claim 4, wherein the mixture is a component of dressings, mayonnaises, margarines, milk products, juices, baked goods and confectionery and the like.

7. The use according to claim 4, wherein the mixture is used for transesterification with hardened fats and for generating lipids having an adjustable solid fat content, melting and crystallization behavior.

8. The use according to claim 4, wherein the mixture is used for parenteral nutrition.

9. The use according to any one of claims 4 to 8, wherein the mixture is used in the form of emulsions with phospholipids or capsules.

10. The use of the mixture according to claim 1 for producing a medicament for treating disorders of fat metabolism.

## Revendications

1. Mélange contenant des lipides de formule (I), 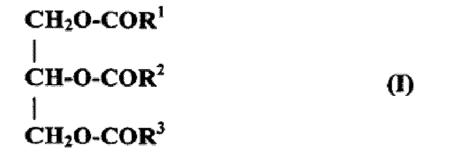 **caractérisé en ce que** R¹CO, R²CO, R³CO sont choisis, indépendamment les uns des autres, dans le groupe constitué par :
(v) un radical acyle saturé linéaire comprenant 6 à 12 atomes de carbone,
(vi) le radical acyle de l'acide linoléique conjugué (CLA)
(vii) le radical acyle d'un acide gras oméga-3 (OF) et
(viii) le radical acyle d'un acide gras oméga-6 (OF),
à condition que la quantité de radicaux acyle CLA dans le mélange représente 3 à 50% en mole et la quantité de radicaux acyle OF dans le mélange représente 5 à 25% en mole - à chaque fois par rapport à la quantité de tous les radicaux acyle dans le mélange.

2. Procédé pour la préparation du mélange selon la revendication 1, **caractérisé en ce qu'**on soumet
(a) ce qu'on appelle des "medium chain triglycerides" (MCT - triglycérides de chaîne moyenne) de formule (II), 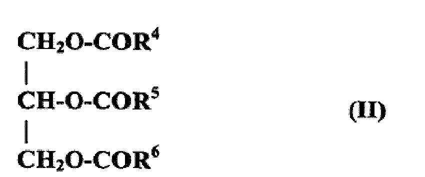 où R⁴CO, R⁵CO et R⁶CO représentent, indépendamment les uns des autres, à chaque fois un radical acyle saturé linéaire comprenant 6 à 12 atomes de carbone, et
(b) de l'acide linoléique conjugué (CLA) et/ou des acides gras oméga-3 (OF) et/ou des acides gras oméga-6 (OF) et/ou des triglycérides à base d'acide linoléique conjugué (TG-CLA) et/ou des triglycérides à base d'acides gras oméga-3 ou d'acides gras oméga-6 (TG-OF) à une transestérification enzymatique.

3. Procédé pour la préparation du mélange selon la revendication 1, **caractérisé en ce qu'**on soumet
(a) des mélanges d'acides gras ou de leurs esters de formule (III),
R⁷COOR⁸ (III)
dans laquelle R⁷CO représente un radical acyle saturé linéaire comprenant 6 à 12 atomes de carbone et R⁸ représente hydrogène ou un radical alkyle comprenant 1 à 4 atomes de carbone, et
(b) de l'acide linoléique (CLA) et/ou des acides gras oméga-3 et/ou oméga-6 à une estérification ou, selon le cas, une transestérification enzymatique.

4. Utilisation du mélange selon la revendication 1 comme aliment, complément alimentaire, préparation pharmaceutique ou aliment pour animaux.

5. Utilisation selon la revendication 4, **caractérisée en ce que** l'aliment est une huile alimentaire ou, selon le cas, une huile alimentaire diététique, une huile de cuisson et de friture et analogues.

6. Utilisation selon la revendication 4, **caractérisée en ce que** le mélange est un constituant de vinaigrettes, de mayonnaises, de margarines, de produits lactés, de jus, de produits de boulangerie et de pâtisseries et analogues.

7. Utilisation selon la revendication 4, **caractérisée en ce qu'**on utilise le mélange pour la transestérification avec des graisses hydrogénées et pour la production de lipides présentant une teneur en graisse hydrogénée, un comportement de fusion et de cristallisation réglables.

8. Utilisation selon la revendication 4, **caractérisée en ce qu'**on utilise le mélange pour la nourriture parentérale.

9. Utilisation selon l'une quelconque des revendications 4 à 8, **caractérisée en ce qu'**on utilise le mélange sous forme d'émulsions avec des phospholipides ou des capsules.

10. Utilisation du mélange selon la revendication 1 pour la préparation d'un médicament destiné au traitement de maladies du métabolisme des lipides.
